# EUROPEAN PATENT SPECIFICATION

(11) **EP 3 424 422 B1**
(45) Date of publication and mention of the grant of the patent: **12.08.2020**
(21) Application number: 17759495.9
(22) Date of filing: 26.01.2017
(51) Int. Cl.: A61B 5/117, A61B 5/02, A61B 5/00, A61B 5/107, A61B 5/0295

(54) **BIOMETRIC INFORMATION MEASUREMENT DEVICE, INDIVIDUAL IDENTIFICATION DEVICE, INDIVIDUAL IDENTIFICATION METHOD, AND INDIVIDUAL IDENTIFICATION PROGRAM**
BIOMETRISCHE INFORMATIONSMESSVORRICHTUNG, INDIVIDUELLE IDENTIFIKATIONSVORRICHTUNG, INDIVIDUELLES IDENTIFIKATIONSVERFAHREN UND INDIVIDUELLES IDENTIFIKATIONSPROGRAMM
DISPOSITIF DE MESURE D'INFORMATIONS BIOMÉTRIQUES, DISPOSITIF D'IDENTIFICATION INDIVIDUELLE, PROCÉDÉ D'IDENTIFICATION INDIVIDUELLE ET PROGRAMME D'IDENTIFICATION INDIVIDUELLE

(30) Priority: 29.02.2016 JP 2016037139
(43) Date of publication of application: 09.01.2019
(73) Proprietor: Omron Healthcare Co., Ltd., Kyoto 617-0002 (JP); Omron Corporation, Kyoto-shi, Kyoto 600-8530 (JP)
(72) Inventor: OTA Yuya, Kyoto-shi Kyoto 600-8530 (JP); WADA Hirotaka, Kyoto-shi Kyoto 600-8530 (JP); KASAI Masaaki, Kyoto-shi Kyoto 600-8530 (JP); KOKUBO Ayako, Kyoto-shi Kyoto 600-8530 (JP)
(74) Representative: Isarpatent
(86) International application number: PCT/JP2017/002792
(87) International publication number: WO 2017/150023

(56) References cited:
- WO-A1-2013/068955
- JP-A- 2002 312 324
- JP-A- 2009 072 407
- JP-A- 2015 052 999
- US-A1- 2014 085 050

## Description

### Technical Field

The present invention relates to a biological information measurement device, a personal identification device, a personal identification method, and a personal identification program.

### Background Art

As a personal authentication technology which allows only a specific person to use a device, a service, or the like, techniques which use biological information, such as fingerprint authentication, iris authentication, and vein authentication are usually known.

Patent Literatures 1 and 2 disclose techniques in which personal authentication is performed by using a pressure signal waveform corresponding to the heart beat, heart sound, respiration, or the like of a living body other than biological information such as the fingerprint, the iris, and the vein.

Patent Literature 1 discloses a technique in which the internal pressure of an air bag attached to the wrist is detected by a pressure sensor, a feature amount of the heart sound waveform, the respiration waveform, or the like is obtained from a detection signal of the pressure sensor, and personal authentication is performed by using the feature amount.

Patent Literature 2 discloses a technique in which the internal pressure of an air bag attached to the wrist is detected by a pressure sensor, and personal authentication is performed by using information of, for example, the frequency of the heart beat that is measured by the pressure sensor every 24 hours or predetermined hours. Patent Literature 3 discloses a wristband with pressure sensor array for optimized physiological measurement.

### Citation List

### Patent Literature

Patent Literature 1: JP-A-2006-346221
Patent Literature 2: WO04/096045
Patent Literature 3: JP-A-2009-072407

### Summary of Invention

### Technical Problem

In the techniques disclosed in Patent Literature 1 and Patent Literature 2, personal authentication is performed by using biological information that is changed in accordance with the pulsation of a living body. Therefore, the biological information is largely changed by the physical condition or motion of a person to be authenticated, and therefore the authentication accuracy is likely to be lowered.

Personal authentication is required in a wide variety of devices. An example of such devices is a biological information measurement device which measures biological information, for example, blood pressure information such as the systolic blood pressure, the diastolic blood pressure, or the pulse pressure, the pulsation information such as the pulse rate, or the heartbeat information such as the heart rate. For example, a biological information measurement device is used in the home of the patient who received a diagnosis in a hospital. When such a utilization mode is assumed, it is important in diagnosis that biological information which is to be measured by the biological information measurement device has been measured from the person to be measured oneself.

Among biological information measurement devices, there is a device which has a pressure sensor that is to be directly contacted to the living body of the person to be measured, which detects a pressure pulse wave by using the pressure sensor, and which calculates biological information based on the detected pressure pulse wave. When, as in the techniques disclosed in Patent Literatures 1 and 2, a technique in which biological information is acquired by using an air bag and the pressure sensor, and personal authentication is performed by using the biological information is applied to such a biological information measurement device, the device is enlarged in size. Moreover, an additional cost for personal authentication is generated. Furthermore, a work dedicated to personal authentication is necessary, and the work is cumbersome.

The invention has been conducted in view of the above circumstances. It is an object of the invention to provide a biological information measurement device, personal identification device, personal identification method, and personal identification program which can easily perform personal authentication at low cost regardless of the physical condition.

### Solution to Problem

The biological information measurement device of the present invention includes: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; a biological information calculating section which calculates biological information based on information of pressure pulse waves which are detected by the pressure detecting elements in a state where the pressure sensor is pressed against the wrist by the pressing mechanism; and a personal identifying section which determines whether the person to be measured is a registered user who is previously registered or not, based on data indicating relationships between information of pressure signals which are detected by the pressure detecting elements of the pressure sensor in a pressed state where the pressure sensor is pressed against the wrist by the pressing mechanism, and positions of the pressure detecting elements in the one direction.

The personal identification device of the present invention includes: an information acquiring section which acquires data from a biological information measurement device including: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; and a biological information calculating section which calculates biological information based on information of pressure pulse waves that are detected by the pressure detecting elements in a state where the pressure sensor is pressed against the wrist by the pressing mechanism, the data indicating relationships between information of pressure signals which are detected by the pressure detecting elements of the pressure sensor in a pressed state where the pressure sensor is pressed against the wrist by the pressing mechanism, and positions of the pressure detecting elements in the one direction; a personal identifying section which determines whether the person to be measured is a registered user who is previously registered or not, based on the data acquired by the information acquiring section; and a transmitting section which transmits a result of the determination performed by the personal identifying section, to the biological information measurement device.

The personal identification method of the present invention is a method which is performed by a biological information measurement device including: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; and a biological information calculating section which calculates biological information based on information of pressure pulse waves that are detected by the pressure detecting elements in a state where the pressure sensor is pressed against the wrist by the pressing mechanism, the personal identification method including: a personal identifying step of determining whether the person to be measured is a registered user who is previously registered or not, based on data indicating relationships between information of pressure signals which are detected by the pressure detecting elements of the pressure sensor in a pressed state where the pressure sensor is pressed against the wrist by the pressing mechanism, and positions of the pressure detecting elements in the one direction.

The personal identification program of the present invention is a program for causing a computer of a biological information measurement device including: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; and a biological information calculating section which calculates biological information based on information of pressure pulse waves that are detected by the pressure detecting elements in a state where the pressure sensor is pressed against the wrist by the pressing mechanism, to execute a personal identifying step of determining whether the person to be measured is a registered user who is previously registered or not, based on data indicating relationships between information of pressure signals which are detected by the pressure detecting elements of the pressure sensor in a pressed state where the pressure sensor is pressed against the wrist by the pressing mechanism, and positions of the pressure detecting elements in the one direction.

The personal identification method of the present invention includes: an information acquiring step of acquiring data from a biological information measurement device including: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; and a biological information calculating section which calculates biological information based on information of pressure pulse waves that are detected by the pressure detecting elements in a state where the pressure sensor is pressed against the wrist by the pressing mechanism, the data indicating relationships between information of pressure signals which are detected by the pressure detecting elements of the pressure sensor in a pressed state where the pressure sensor is pressed against the wrist by the pressing mechanism, and positions of the pressure detecting elements in the one direction; a personal identifying step of determining whether the person to be measured is a registered user who is previously registered or not, based on the data acquired in the information acquiring step; and a transmitting step of transmitting a result of the determination performed in the personal identifying step, to the biological information measurement device.

The personal identification program of the present invention is a program for causing a computer to execute: an information acquiring step of acquiring data from a biological information measurement device including: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; and a biological information calculating section which calculates biological information based on information of pressure pulse waves that are detected by the pressure detecting elements in a state where the pressure sensor is pressed against the wrist by the pressing mechanism, the data indicating relationships between information of pressure signals which are detected by the pressure detecting elements of the pressure sensor in a pressed state where the pressure sensor is pressed against the wrist by the pressing mechanism, and positions of the pressure detecting elements in the one direction; a personal identifying step of determining whether the person to be measured is a registered user who is previously registered or not, based on the data acquired in the information acquiring step; and a transmitting step of transmitting a result of the determination performed in the personal identifying step, to the biological information measurement device.

### Advantageous Effects of Invention

According to the invention, it is possible to provide a biological information measurement device, personal identification device, personal identification method, and personal identification program which can easily perform personal authentication at low cost regardless of the physical condition.

### Brief Description of Drawings

[Fig. 1] Fig. 1 is a diagram schematically showing the configuration of the appearance of a biological information measurement device 100 for illustrating an embodiment of the invention.
[Fig. 2] Fig. 2 is a plan diagram as viewing a pressure sensor 10 of the biological information measurement device 100 shown in Fig. 1, from the side of a contact plane with the wrist.
[Fig. 3] Fig. 3 is a block diagram showing the internal configuration of a body unit 1 of the biological information measurement device 100 shown in Fig. 1.
[Fig. 4] Fig. 4 is a functional block diagram of a controller 30 shown in Fig. 3.
[Fig. 5] Fig. 5 is a sectional diagram showing a state where the pressure sensor 10 of the biological information measurement device 100 shown in Fig. 1 is pressed against the wrist.
[Fig. 6] Fig. 6 is a view showing an example of a tonogram in the case where the person to be measured is an adult male (a person in whom the length around the wrist is long or has a first value).
[Fig. 7] Fig. 7 is a view showing an example of a tonogram in the case where the person to be measured is an adult female (a person in whom the length around the wrist has a second value which is smaller than the first value).
[Fig. 8] Fig. 8 is a view showing an example of a tonogram in the case where the person to be measured is a child (a person in whom the length around the wrist has a value which is smaller than the second value).
[Fig. 9] Fig. 9 is a flowchart illustrating the operation of the biological information measurement device 100 shown in Fig. 1.
[Fig. 10] Fig. 10 is a flowchart showing in detail a process of step S2 shown in Fig. 9.
[Fig. 11] Fig. 11 is a flowchart illustrating a first modification of the process of step S2 shown in Fig. 9.
[Fig. 12] Fig. 12 is a flowchart illustrating a second modification of the process of step S2 shown in Fig. 9.
[Fig. 13] Fig. 13 is a flowchart illustrating a modification of the operation of the biological information measurement device 100 shown in Fig. 1.
[Fig. 14] Fig. 14 is a diagram schematically showing the configuration of a personal authentication system 300 including a personal identification device 200 of an embodiment of the invention.
[Fig. 15] Fig. 15 is a diagram showing the internal configuration of a body unit of a biological information measurement device 100A shown in Fig. 14.
[Fig. 16] Fig. 16 is a functional block diagram of a controller 30A of the biological information measurement device 100A shown in Fig. 15.
[Fig. 17] Fig. 17 is a functional block diagram of the personal identification device 200 shown in Fig. 14.

### Description of Embodiments

Hereinafter, an embodiment of the invention will be described with reference to the drawings.

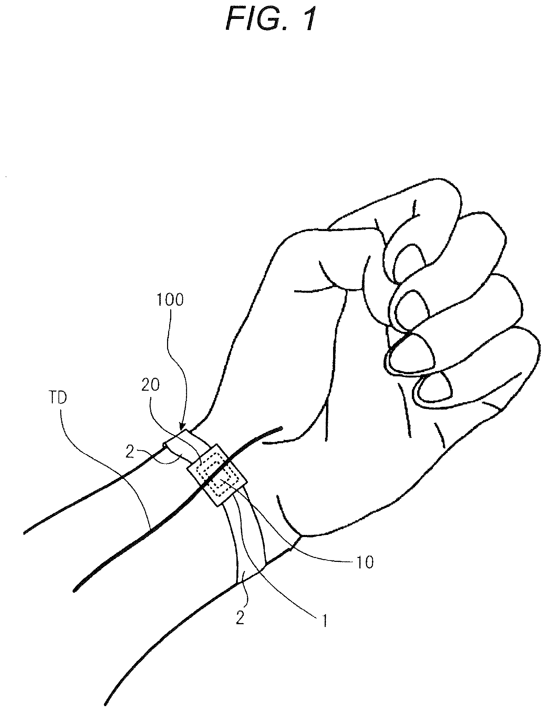

Fig. 1 is a diagram schematically showing the configuration of the appearance of a biological information measurement device 100 for illustrating the embodiment of the invention.

The biological information measurement device 100 includes a body unit 1 and a belt 2 fixed to the body unit 1. The biological information measurement device 100 is used while being attached to the wrist in which the radial artery TD that is the target of measurement of biological information exists under the skin, or used while the body unit 1 is attached to the wrist by the belt 2. Biological information which is the target of measurement by the biological information measurement device 100 is blood pressure information such as the systolic blood pressure, the diastolic blood pressure, or the pulse pressure, the pulsation information such as the pulse rate, or the heartbeat information such as the heart rate.

The body unit 1 of the biological information measurement device 100 includes a pressure sensor 10 which detects a pressure pulse wave from the radial artery TD, and a pressing mechanism 20 for pressing the pressure sensor 10 against the wrist.

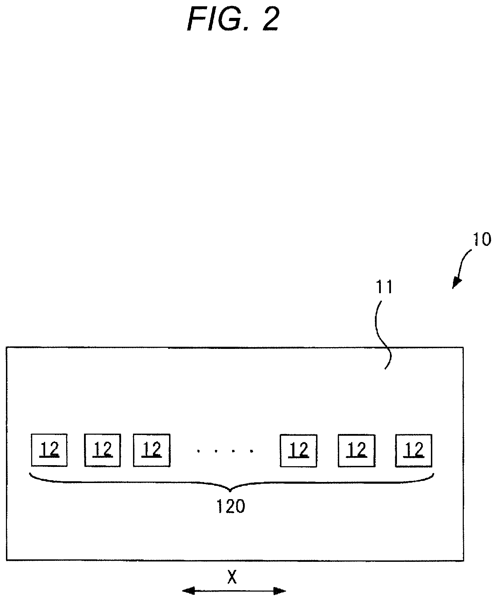

Fig. 2 is a plan diagram as viewing the pressure sensor 10 of the biological information measurement device 100 shown in Fig. 1, from the side of a contact plane with the wrist. As shown in Fig. 2, the pressure sensor 10 has an element row 120 which is formed on a planar substrate 11.

The element row 120 is configured by a plurality of pressure detecting elements 12 which are arranged in a direction X that is the one direction. As the pressure detecting elements 12, any kind of elements which detect a pressure, and which convert the pressure to an electric signal may be available, and, for example, elements utilizing the piezoresistance effect may be used.

The intervals of the plurality of pressure detecting elements 12 in the arrangement direction are made sufficiently small so that a necessary and sufficient number of elements can be arranged above the radial artery TD. The distance between the pressure detecting elements which are at the respective ends of the plurality of pressure detecting elements 12 is necessarily and sufficiently made larger than the diameter of the radial artery TD.

The pressure sensor 10 is pressed against the wrist by the pressing mechanism 20 in a state where the direction X that is the arrangement direction of the plurality of pressure detecting elements 12 included in the element row 120 intersects with the elongation direction of the radial artery TD. Alternatively, the pressure sensor 10 may have a configuration where a plurality of element rows 120 are arranged on the substrate 11 in a direction perpendicular to the direction X.

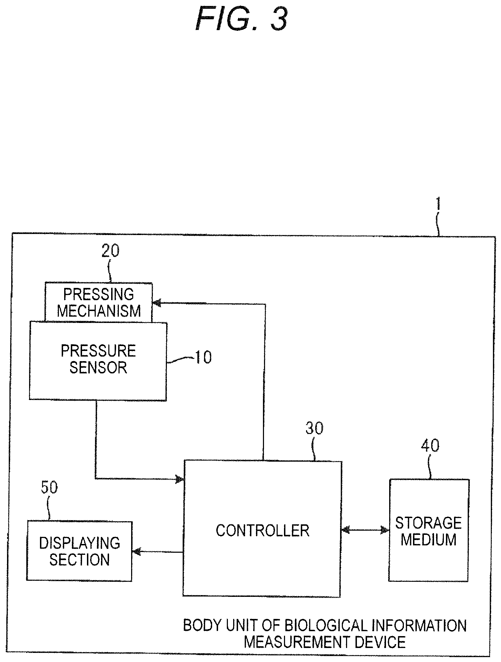

Fig. 3 is a block diagram showing the internal configuration of the body unit 1 of the biological information measurement device 100 shown in Fig. 1.

The body unit 1 includes the pressure sensor 10, the pressing mechanism 20, a controller 30 which generally controls the entire, a storage medium 40, and a displaying section 50.

The pressing mechanism 20 is configured by, for example, an air bag that is fixed to the surface opposite to the surface of the substrate 11 on which the element row 120 is formed, and a pump for adjusting the internal pressure of the air bag. The pressing force (the internal pressure of the pump) which is applied to the wrist by the pressing mechanism 20 is controlled by the controller 30. The pressing mechanism 20 may be configured any kind of device as far as it can press the pressure sensor 10 against the wrist, and is not limited to a configuration in which an air bag is used.

The pressure sensor 10 supplies pressure signals which are detected by the pressure detecting elements 12 constituting the element row 120, to the controller 30.

The controller 30 includes a ROM (Read Only Memory), a RAM (Random Access Memory), and a processor, and generally controls the entire body unit 1 by execution of programs stored in the ROM by the processor. The programs include the personal identification program. The RAM functions as a work memory in the case where the controller 30 performs various processes.

The storage medium 40 is a medium in and from which data can be stored and read, and, for example, a flash memory may be used as the medium. The storage medium 40 may be of the mobile type such as a memory card, or fixed to the body unit 1 so as to be undetachable.

The displaying section 50 is used for displaying various kinds of information containing biological information, and configured by a liquid crystal display device or the like.

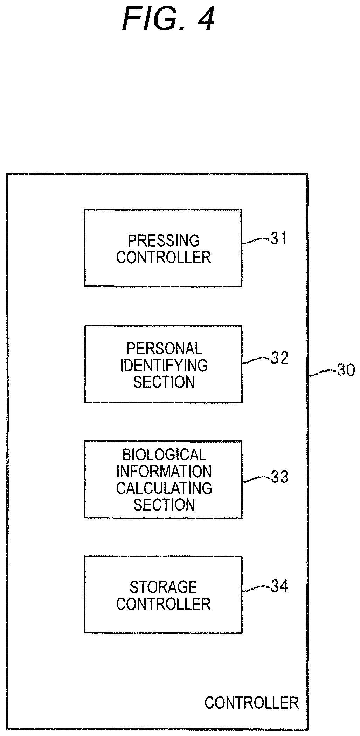

Fig. 4 is a functional block diagram of the controller 30 shown in Fig. 3.

The controller 30 executes programs to function as a pressing controller 31, a personal identifying section 32, a biological information calculating section 33, and a storage controller 34.

The pressing controller 31 drives the pressing mechanism 20 to control the pressing force of the pressure sensor 10 against the wrist exerted by the pressing mechanism 20.

The pressing controller 31 performs a pressing force increasing control in which, at a predetermined timing such as a timing when instructions for measuring biological information is issued to the biological information measurement device 100, the pressing force exerted by the pressing mechanism 20 is increased from the initial value to a previously set value (a value at which a pressure signal can be detected from the radius and the tendon). The set value is set to a value which is higher than the optimum pressing force (a pressing force at which the tonometry state is obtained) that is adequate for detecting a pressure pulse wave.

The personal identifying section 32 determines whether the person to be measured who is using the biological information measurement device 100 is a registered user who is previously registered in the biological information measurement device 100 or not, based on data indicating relationships between information of the pressure signals which are detected by the pressure detecting elements 12 in the state where the pressure sensor 10 is pressed against the wrist by the pressing mechanism 20, and the positions of the pressure detecting elements 12 in the direction X.

The information of each pressure signal is the absolute value of the pressure signal, the amplitude of an AC component (an AC component which is produced by a pulse wave) contained in the pressure signal, the mean value of the maximum and minimum values of the AC component contained in the pressure signal, or the like. Hereinafter, description will be made with the assumption that the information of each pressure signal is the absolute value of the pressure signal.

The biological information calculating section 33 calculates biological information by using a well known method, based on the information of the pressure pulse wave which is detected by the optimum pressure detecting element of the plurality of pressure detecting elements 12 constituting the element row 120, in a state where the pressure sensor 10 is pressed against the wrist with the optimum pressing force by the pressing mechanism 20.

In the specification, the information of the pressure pulse wave is information specifying the shape of the pressure pulse wave such as the amplitude of the pressure pulse wave, the maximum value of the pressure pulse wave (the absolute value of the pressure signal), or the minimum value of the pressure pulse wave. The amplitude of the pressure pulse wave is a value which is obtained by subtracting the minimum value of the pressure pulse wave from the maximum value.

The optimum pressing force is a pressing force which realizes a state where a pressure pulse wave can be detected from the radial artery TD that is pressed with the optimum pressing force, without being affected by the tension in the circumferential direction of the blood vessel, i.e., the tonometry state. The optimum pressure detecting element is the pressure detecting element 12 which is located directly above a portion of the radial artery TD that is pressed and flattened with the optimum pressing force by the pressure sensor 10.

The storage controller 34 controls a process of storing biological information calculated by the biological information calculating section 33, in the storage medium 40.

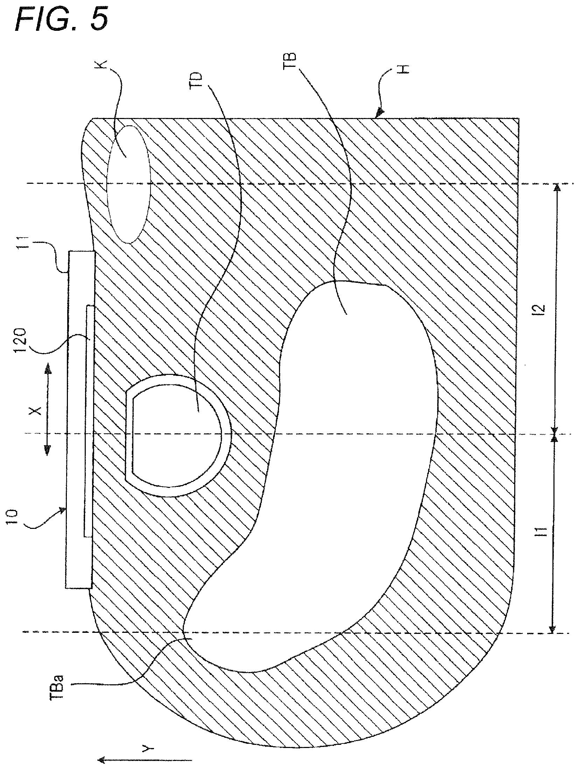

Fig. 5 is a sectional diagram showing a state where the pressure sensor 10 of the biological information measurement device 100 shown in Fig. 1 is pressed against the wrist. Fig. 5 is a sectional diagram in a state where the left elbow of the person to be measured is on the front side of the sheet, the left hand of the person to be measured is on the back side of the sheet, and the palm of the person to be measured is directed in the direction Y in the figure.

In Fig. 5, shown are the wrist H of the person to be measured, the pressure sensor 10 which is pressed against the wrist H, the radial artery TD in the wrist H, the radius TB in the wrist H and the styloid process TBa of the radius TB, and the tendon K in the wrist H.

Here, the tonogram which can be produced from data showing relationships between the absolute values of the pressure signals which are detected by the pressure detecting elements 12 in the pressed state where the pressure sensor 10 is pressed against the wrist by the pressing mechanism 20, and the positions of the pressure detecting elements 12 in the direction X will be described.

The tonogram in the specification is a graph which is expressed by setting information of the pressure signals that are detected by the pressure detecting elements 12 in the pressed state where the pressure sensor 10 is pressed against the wrist by the pressing mechanism 20, as a first axis (for example, the ordinate), and setting the positions of the pressure detecting elements 12 in the direction X as a second axis (for example, the abscissa). The tonogram may be a graph in which the first axis is the abscissa, and the second axis is the ordinate.

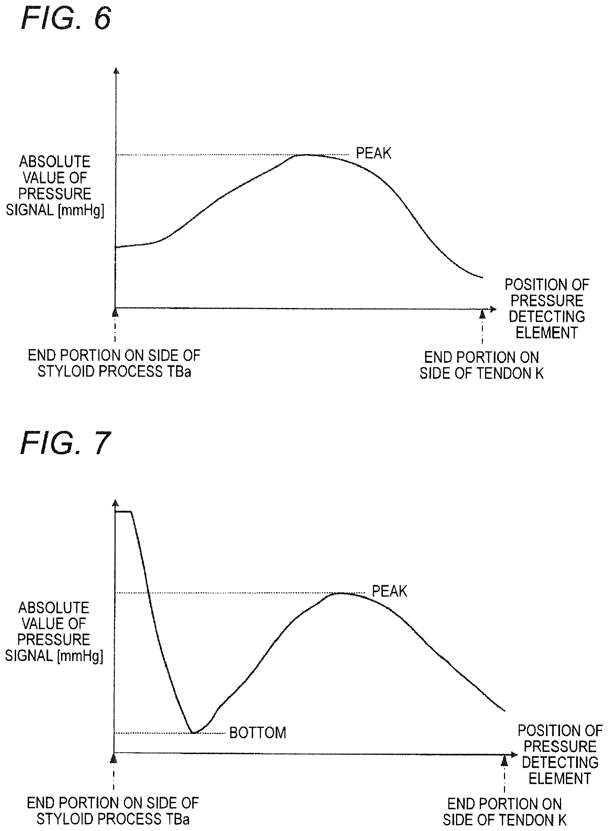

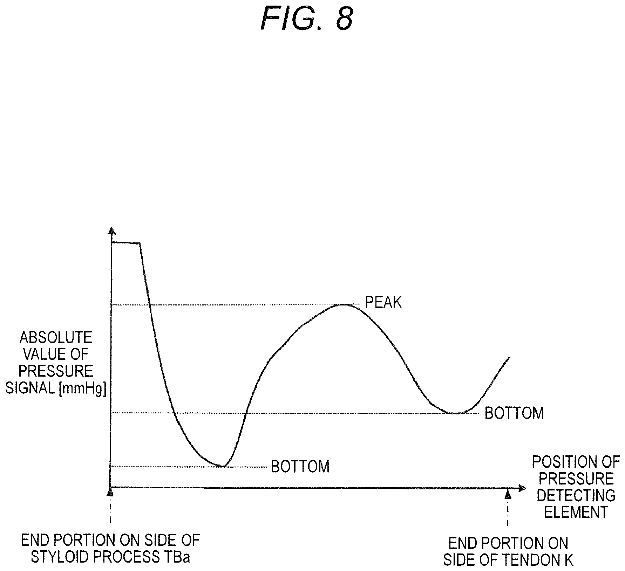

Fig. 6 is a view showing an example of a tonogram in the case where the person to be measured is an adult male (a person in whom the length around the wrist is long or has a first value). Fig. 7 is a view showing an example of a tonogram in the case where the person to be measured is an adult female (a person in whom the length around the wrist has a second value which is smaller than the first value). Fig. 8 is a view showing an example of a tonogram in the case where the person to be measured is a child (a person in whom the length around the wrist has a value which is smaller than the second value). Figs. 6 to 8 show tonograms which are obtained in a state where the pressing force is a set value.

In each of Figs. 6, 7, and 8, the abscissa indicates the position of each of the pressure detecting elements 12 in the direction X. The left end of the each of the abscissas of Figs. 6, 7, and 8 indicates the position of the pressure detecting element 12 which is one of the plurality of pressure detecting elements 12 constituting the element row 120, and which is in the end portion on the side of the styloid process TBa of the radius TB.

The ordinate in each of Figs. 6, 7, and 8 indicates the absolute value of the pressure signal which is detected by each of the pressure detecting elements 12.

As seen from the graphs of Figs. 6, 7, and 8, the shape of the graph showing the tonogram is changed depending on the length around the wrist of the person to be measured. This is because positional relationships of the radial artery TD, styloid process TBa, and tendon K which are shown in Fig. 5 are made different by the difference in the length around the wrist of the person to be measured. Depending of the difference in physical size of the person to be measured, namely, at least one of a distance 11 between the radial artery TD and styloid process TBa shown in Fig. 5, in the direction X, and a distance 12 between the radial artery TD and the tendon K in the direction X is changed.

When the length around the wrist is short, and the distance 11 is shorter than that in the state shown in Fig. 5, for example, the styloid process TBa approaches a region below the element row 120. Therefore, the pressure due to the styloid process TBa is detected by the element row 120, and the tonogram is in the state shown in Fig. 7.

When the length around the wrist is short, and each of the distance 11 and the distance 12 is shorter than that in the state shown in Fig. 5, moreover, the styloid process TBa and the tendon K approach a region below the element row 120. Therefore, the pressures due to the styloid process TBa and the tendon K are detected by the element row 120, and the tonogram is in the state shown in Fig. 8.

When the person to be measured has a standard body shape, for example, it is statistically known that the length around the wrist of the person to be measured can be classified into three classes depending on the body height which is an element for determining the physical size.

Specifically, the length around the wrist of a person whose body height exceeds 165 cm is longer than 15.9 cm, that of a person whose body height is equal to or longer than 157 cm and equal to or shorter that 165 cm is equal to or longer than 15.2 cm and equal to or shorter than 15.9, and that of a person whose body height is shorter than 157 cm is equal to or longer than 14 cm and equal to or shorter than 14.6 cm.

The biological information measurement device 100 is designed so that, in the case where the length around the wrist exceeds 15.9 cm, a tonogram that is obtained in a state where the wrist is pressed by the pressure sensor 10 while setting the pressing force to the above-described set value exhibits a shape which has one peak as shown in Fig. 6.

Moreover, the biological information measurement device 100 is designed so that, in the case where the length around the wrist is equal to or longer than 15.2 cm and equal to or shorter than 15.9, a tonogram that is obtained in a state where the wrist is pressed by the pressure sensor 10 while setting the pressing force to the above-described set value exhibits a shape which has one peak and one bottom as shown in Fig. 7.

Furthermore, the biological information measurement device 100 is designed so that, in the case where the length around the wrist is equal to or longer than 14 cm and equal to or shorter than 14.6 cm, a tonogram that is obtained in a state where the wrist is pressed by the pressure sensor 10 while setting the pressing force to the above-described set value exhibits a shape which has one peak and two bottoms as shown in Fig. 8.

A peak of a tonogram is a portion where the absolute value of the pressure signal changes from increasing to decreasing when the absolute value of the pressure signal is seen while advancing from the end portion on the side of the styloid process TBa toward the end portion on the side of the tendon K.

Moreover, a bottom of a tonogram is a portion where the absolute value of the pressure signal changes from decreasing to increasing when the absolute value of the pressure signal is seen while advancing from the end portion on the side of the styloid process TBa toward the end portion on the side of the tendon K.

The storage medium 40 of the biological information measurement device 100 stores identification information for identifying the person to be measured in whom the use of the biological information measurement device 100 is requested by the doctor or the like. In the biological information measurement device 100, a user registration mode in which the identification information is produced and stored in the storage medium 40 is disposed.

Hereinafter, the operation of the biological information measurement device 100 when the user registration mode is set will be described.

When the biological information measurement device 100 is attached to the wrist of the person to be measured, and instructions for user registration is given by a button operation or the like, the controller 30 causes the pressing mechanism 20 to start pressing of the pressure sensor 10, and data indicating relationships between the absolute values of the pressure signals detected by the pressure detecting elements 12 in a state where the pressing force is increased to the above-described set value, and the positions of the pressure detecting elements 12 in the direction X are stored in the RAM.

Based on the data stored in the RAM, then, the controller 30 analyzes a tomogram indicating the data to detect a peak(s) and bottom(s) of the tomogram, stores information of the number of the detected peaks and that of the detected bottoms as the identification information in the storage medium 40, and ends the user registration mode. As a result of the series of processes, the person to be measured is registered as the registered user in the biological information measurement device 100.

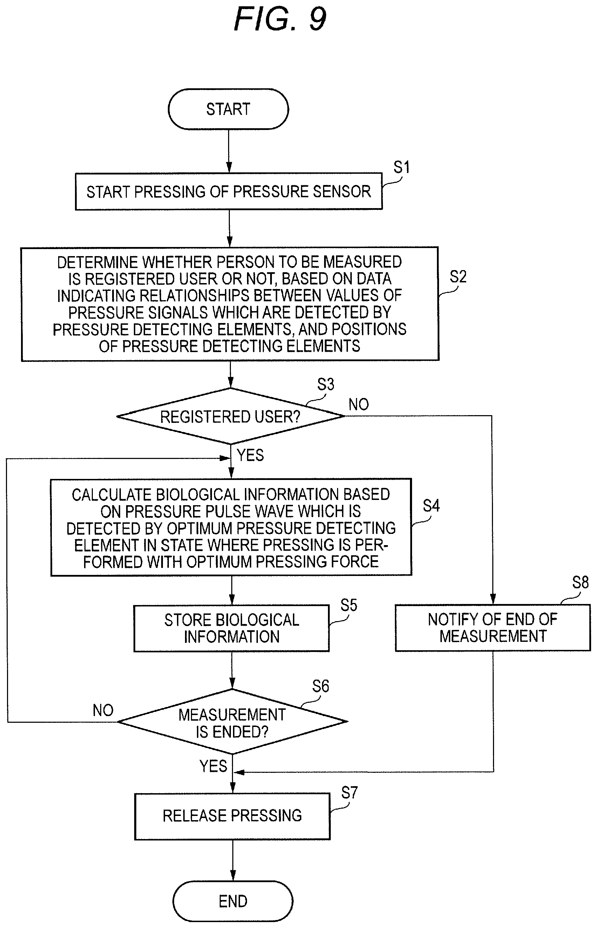

Fig. 9 is a flowchart illustrating the operation of the biological information measurement device 100 shown in Fig. 1.

When the person to be measured attaches the biological information measurement device 100 to the wrist, and presses a measurement start button which is disposed in the biological information measurement device 100, and which is not shown, the flow shown in Fig. 9 is started.

First, the pressing controller 31 performs a pressing force increasing control in which the pressing force that is applied to the wrist by the pressing mechanism 20 is increased from the initial value to the set value (step S1).

Next, the personal identifying section 32 determines whether the person to be measured is the registered user or not, based on data (tonogram) indicating relationships between the absolute values of the pressure signals which are detected by the pressure detecting elements 12 of the pressure sensor 10 in a state where the pressing force is at the set value, and the positions of the pressure detecting elements 12 in the direction X (step S2).

If it is determined that the person to be measured is not the registered user (step S3: NO), the personal identifying section 32 causes the displaying section 50 to display information indicating that the measurement is ended, and also a reason for ending the measurement (step S8). After step S8, the pressing controller 31 returns the pressing force to the initial value (= 0) (step S7), and the measurement operation is ended.

If it is determined that the person to be measured is the registered user (step S3: YES), the controller 30 determines the optimum pressing force and the optimum pressure detecting element based on information of the pressure pulse wave which is detected by the pressure sensor 10 until the pressing force reaches the above-described set value. Then, the pressing controller 31 causes the pressure sensor 10 to be pressed against the wrist with the optimum pressing force, and the biological information calculating section 33 calculates biological information based on the pressure pulse wave which, in this state, is detected by the optimum pressure detecting element (step S4). Thereafter, the storage controller 34 causes the biological information which is calculated in step S4, to be stored in the storage medium 40 (step S5).

After step S5, the processes of step S4 and step S5 are repeatedly performed until instructions for ending the measurement of biological information is given. When instructions for ending the measurement of biological information is issued after step S5 (step S6: YES), the pressing controller 31 returns the pressing force to the initial value (= 0) (step S7), and the measurement operation is ended.

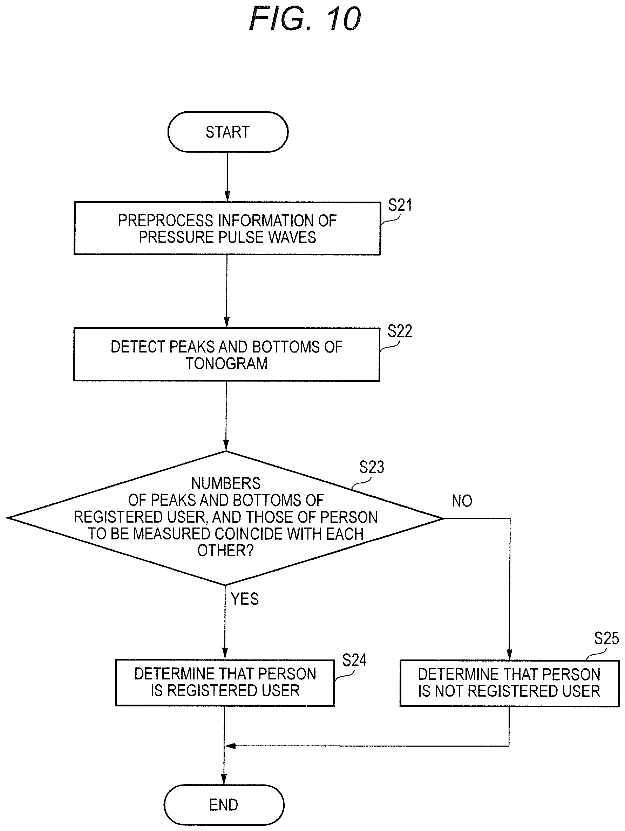

Fig. 10 is a flowchart showing in detail the process of step S2 shown in Fig. 9.

First, the personal identifying section 32 applies preprocessing to the absolute values of the pressure signals constituting the tonogram (step S21). The preprocessing is a process for removing noise components contained in the absolute values of the pressure signals, and fine variation between adjacent pressure signals. As a result of the preprocessing, a peak(s) and a bottom(s) can be easily detected from the tonogram.

Next, the personal identifying section 32 analyzes the preprocessed tonogram to detect a peak(s) and bottom(s) of the tonogram (step S22).

The personal identifying section 32 determines whether the number of peaks of the tonogram detected in step S22 coincides with that of peaks constituting the identification information stored in the storage medium 40 or not, and the number of bottoms of the tonogram detected in step S22 coincides with that of bottoms constituting the identification information stored in the storage medium 40 or not (step S23)

If the determination in step S23 is YES, the personal identifying section 32 determines that the person to be measured is the registered user (step S24), and, if the determination in step S23 is NO, determines that the person to be measured is not the registered user (step S25).

According to the biological information measurement device 100, as described above, it is possible to determine whether the person to be measured is the registered user or not, based on a feature amount (the number of bottoms and that of peaks) of a tonogram which is produced from the absolute values of pressure signals detected from the person to be measured. The positions of bones and tendons are not changed depending on the physical condition of the person to be measured, and therefore the numbers of peaks and bottoms of a tonogram are not changed depending on the physical condition of the person to be measured. Therefore, personal identification can be performed with stable accuracy regardless of the physical condition of the person to be measured. In the biological information measurement device 100, moreover, personal identification can be performed by using the pressure sensor 10 which is used for calculating biological information, and therefore hardware dedicated to personal identification is not necessary. Consequently, the production cost of the biological information measurement device 100 can be prevented from being increased.

According to the biological information measurement device 100, moreover, the optimum pressing force and the optimum pressure detecting element can be determined during the process of pressing the pressure sensor 10 in order to perform personal identification. Therefore, the pressing control for personal identification, and the process of determining the optimum pressing force and optimum pressure detecting element which are necessary for calculating biological information can be performed in parallel. The time period elapsed until the start of measurement of biological information can be shortened. When the pressing control is conducted one time, furthermore, the personal identification, and the determinations of the optimum pressing force and the optimum pressure detecting element can be performed, and therefore the power consumption can be reduced.

In the above description, it is assumed that the identification information is the numbers of peaks and bottoms of a tonogram. In a modification, the identification information may be one of information of the distance between the position of the pressure detecting element 12 corresponding to the peak of the tonogram, and that of the pressure detecting element 12 corresponding to the bottom of the tonogram on the side of the styloid process TBa, and information of the distance between the position of the pressure detecting element 12 corresponding to the peak of the tonogram, and that of the pressure detecting element 12 which is among the plurality of pressure detecting elements 12 of the pressure sensor 10, and which is in the end portion on the side of the styloid process TBa.

In this case, when the biological information measurement device 100 is attached to the wrist of the person to be measured, and instructions for performing the user registration is given by operating a button or the like, the controller 30 causes the pressing mechanism 20 to start pressing of the pressure sensor 10, and causes data indicating relationships between the absolute values of the pressure signals which are detected by the pressure detecting elements 12 in a state where the pressing force reaches the above-described set value, and the positions of the pressure detecting elements 12 in the direction X, to be stored in the RAM.

Based on the above-described data stored in the RAM, next, the controller 30 detects a peak(s) and bottom(s) of a tonogram indicating the data. In the case where bottoms can be detected from the tonogram as shown in Fig. 7 or 8, then, the controller 30 calculates first information of the distance between the position of the pressure detecting element 12 corresponding to the detected peak of the tonogram, and that of the pressure detecting element 12 corresponding to the bottom which is the one of the detected bottoms, and which is on the side of the styloid process TBa, causes the calculated first distance information to be stored as identification information in the storage medium 40, and ends the user registration mode.

By contrast, in the case where a bottom cannot be detected from the tonogram as shown in Fig. 6, the controller 30 calculates second information of the distance between the position of the pressure detecting element 12 corresponding to the detected peak of the tonogram, and that of the pressure detecting element 12 which is among the plurality of pressure detecting elements 12 of the pressure sensor 10, and which is in the end portion of on the side of the styloid process TBa, causes the calculated second distance information to be stored as identification information in the storage medium 40, and ends the user registration mode.

The position of the pressure detecting element 12 corresponding to the peak of the tonogram corresponds to the position directly above the center position of the radial artery TD in the direction X. Therefore, the first distance information and second distance information which are obtained as described above correspond respectively to the distance between the radial artery TD of the person to be measured and the styloid process TBa of the radius in the direction X. The distance is different depending on the person to be measured, and therefore can be used as identification information.

In some cases, a plurality of pressure detecting elements 12 corresponding to the peak of the tomogram are detected. In such cases, the position of the pressure detecting element 12 which is closest to the middle among the plurality of pressure detecting elements 12 can be handled as the position of the pressure detecting element 12 corresponding to the peak of the tonogram.

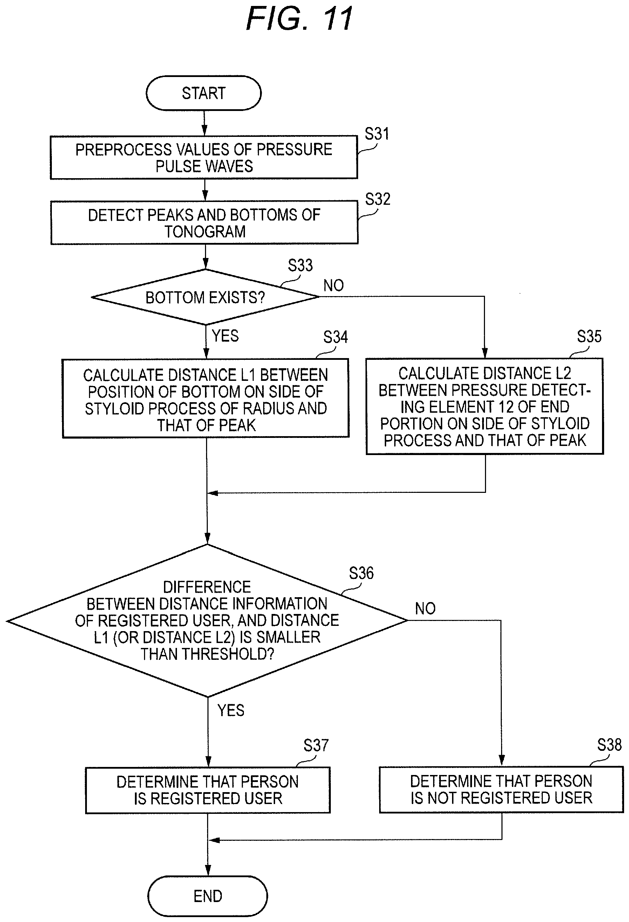

Fig. 11 is a flowchart illustrating a first modification of the process of step S2 shown in Fig. 9. In the first modification, it is assumed that one of the first distance information and the second distance information is previously stored in the storage medium 40 as identification information.

First, the personal identifying section 32 applies preprocessing to the absolute values of the pressure signals constituting the tonogram (step S31). The preprocessing is a process for removing noise components contained in the absolute values of the pressure signals, and fine variation between adjacent pressure signals. As a result of the preprocessing, a peak(s) and a bottom(s) can be easily detected from the tonogram.

Next, the personal identifying section 32 analyzes the preprocessed tonogram to detect a peak(s) and bottom(s) of the tonogram (step S32).

If a bottom(s) can be detected from the tonogram (step S33: YES), the personal identifying section 32 calculates a distance L1 which is the above-described first distance information (step S34). If a bottom(s) cannot be detected from the tonogram (step S33: NO), the personal identifying section 32 calculates a distance L2 which is the above-described second distance information (step S35).

After the distance L1 or the distance L2 is calculated, the personal identifying section 32 determines whether the difference between the distance information of the registered user stored in the storage medium 40, and the distance L1 or the distance L2 is smaller than a threshold or not (step S36).

If the determination in step S36 is YES, the personal identifying section 32 determines that the person to be measured is the registered user (step S37), and, if the determination in step S36 is NO, determines that the person to be measured is not the registered user (step S38).

Also in the first modification, it is possible to attain the above-described effects.

When the personal identification process shown in Fig. 10, and that shown in Fig. 11 are combined with each other, it is possible to improve the accuracy of the personal identification.

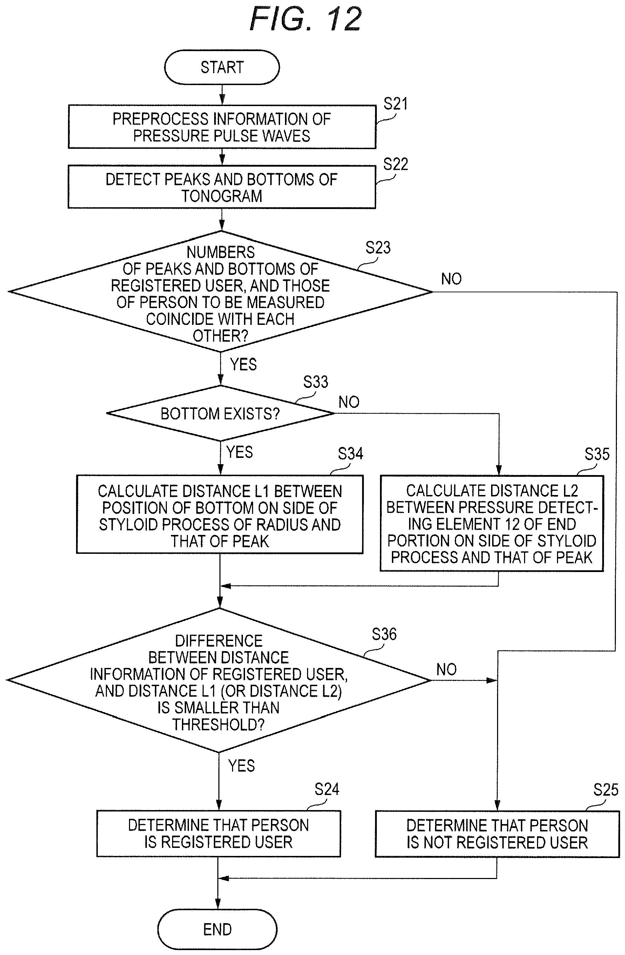

Fig. 12 is a flowchart illustrating a second modification of the process of step S2 shown in Fig. 9. In the second modification, it is assumed that one of the first distance information and the second distance information, and information of the numbers of peaks and bottoms of the tonogram are previously stored as the identification information in the storage medium 40. In Fig. 12, the processes identical with those shown in Figs. 10 and 11 are denoted by the same reference numerals, and their description is omitted.

In the process example shown in Fig. 12, first, the processes of steps S21 to S23 are performed, and, if the determination in step S23 is NO, the process of step S25 is performed. If the determination in step S23 is YES, the processes of steps S33 to S36 are performed. Then, if the determination in step S36 is NO, the process of step S25 is performed, and, if the determination in step S36 is YES, the process of step S24 is performed.

According to the process of the second modification, personal identification can be performed with high accuracy. In Fig. 12, the processes of steps S33 to S36 may be performed after step S22, and, if the determination in step S36 is YES, the process of step S23 may be performed.

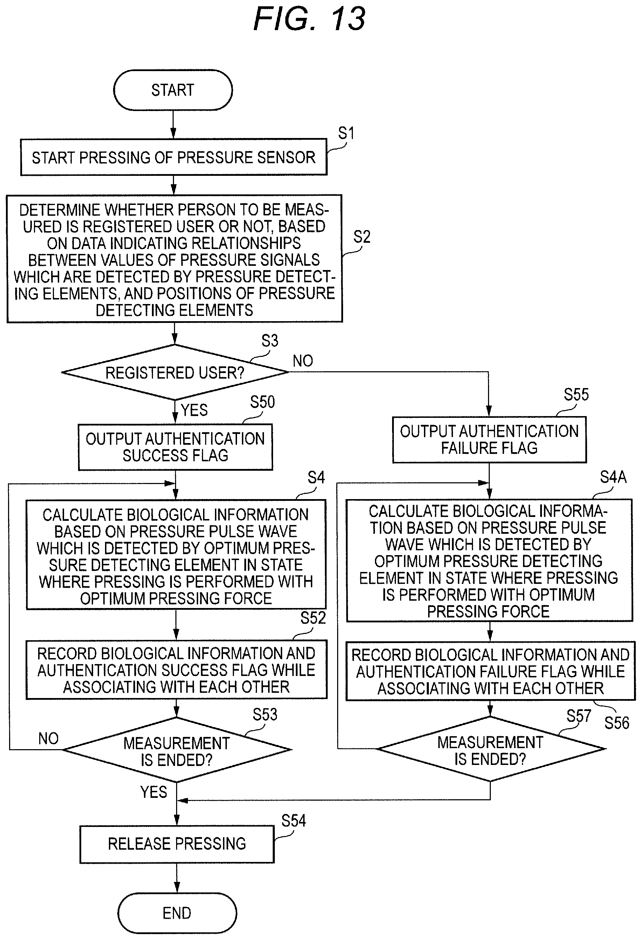

Fig. 13 is a flowchart illustrating a modification of the operation of the biological information measurement device 100 shown in Fig. 1. In Fig. 13, the processes identical with those shown in Fig. 9 are denoted by the same reference numerals, and their description is omitted.

If the determination in step S3 is YES, the personal identifying section 32 supplies an authentication success flag indicating that the person to be measured is the registered user, to the storage controller 34 (step S50). After step S50, the process of step S4 is performed, and biological information is calculated. After step S4, the storage controller 34 causes the authentication success flag which is supplied in step S50 from the personal identifying section 32, and the biological information which is calculated in step S4, to be stored in the storage medium 40 while associating with each other (step S52).

After step S52, the processes of step S4 and step S52 are repeatedly performed until instructions for ending the measurement of biological information is given. When instructions for ending the measurement of biological information is issued after step S52 (step S53: YES), the pressing controller 31 returns the pressing force to the initial value (= 0) (step S54), and the measurement operation is ended.

If the determination in step S3 is NO, the personal identifying section 32 supplies an authentication failure flag indicating that the person to be measured is not the registered user, to the storage controller 34 (step S55). After step S55, the process of step S4A the content of which is identical with that of step S4 is performed, and biological information is calculated. After step S4A, the storage controller 34 causes the authentication failure flag which is supplied in step S55, and the biological information which is calculated in step S4A, to be stored in the storage medium 40 while associating with each other (step S56).

After step S56, the processes of step S4A and step S56 are repeatedly performed until instructions for ending the measurement of biological information is given. When instructions for ending the measurement of biological information is issued after step S56 (step S57: YES), the pressing controller 31 returns the pressing force to the initial value (= 0) (step S54), and the measurement operation is ended.

According to the operation example shown in Fig. 13, as described above, measurement of biological information can be performed on also a person who is not the registered user, and the convenience is improved. With respect to biological information of a person who is not the registered user, the authentication failure flag is stored while associated therewith. Even in the case where the doctor or the like checks biological information stored in the storage medium 40, therefore, biological information of the registered user can be managed separately from that of a person who is not the registered user.

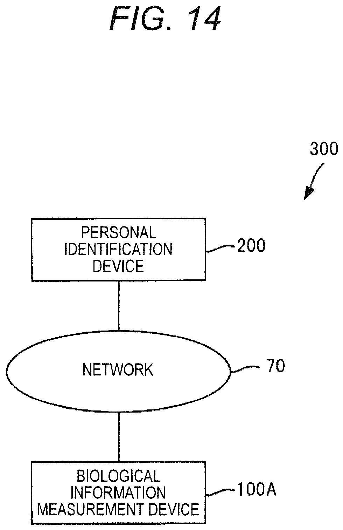

Fig. 14 is a diagram schematically showing the configuration of a personal authentication system 300 including a personal identification device 200 of an embodiment of the invention.

The personal authentication system 300 includes a biological information measurement device 100A and the personal identification device 200. The biological information measurement device 100A and the personal identification device 200 are connected to a network 70 such as the Internet, and mutually communicable through the network 70.

The biological information measurement device 100A is identical in appearance with the biological information measurement device 100 shown in Fig. 1, and includes a body unit and a belt. The personal identification device 200 is a server or an electronic device having a processor, such as a personal computer.

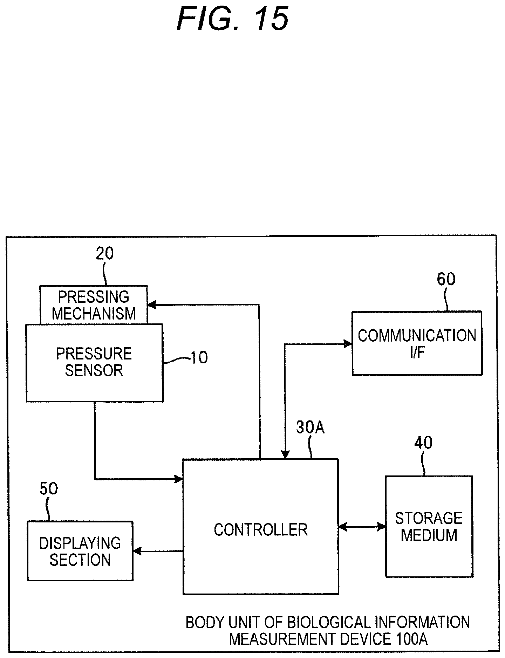

Fig. 15 is a diagram showing the internal configuration of the body unit of the biological information measurement device 100A shown in Fig. 14. The internal configuration of the body unit of the biological information measurement device 100A is identical with that of the body unit of the biological information measurement device 100 except that a communication interface (I/F) 60 which is connected to the network 70 to communicate with an external apparatus is added, and the controller 30 is changed to a controller 30A.

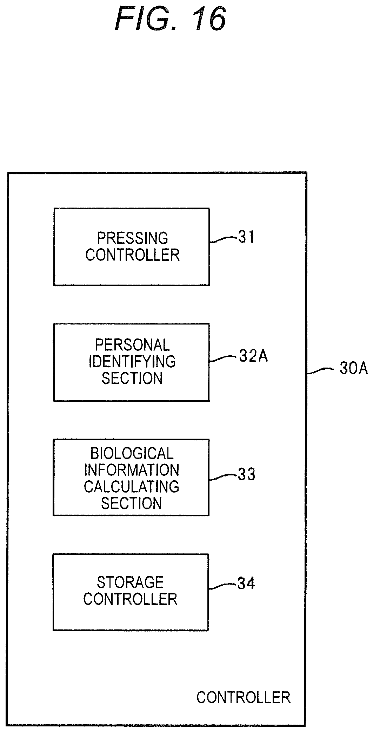

Fig. 16 is a functional block diagram of the controller 30A of the biological information measurement device 100A shown in Fig. 15. The functional block of the controller 30A is identical with that of the controller 30 of the biological information measurement device 100 except that the personal identifying section 32 is changed to a personal identifying section 32A.

The personal identifying section 32A produces data (data of the above-described tonogram) indicating relationships between information of the pressure signals which are detected by the pressure detecting elements 12 in the state where the pressure sensor 10 is pressed against the wrist by the pressing mechanism 20, and the positions of the pressure detecting elements 12 in the direction X, and transmits the data from the communication I/F 60 to the personal identification device 200. The personal identifying section 32A acquires information of a personal identification result which is transmitted from the personal identification device 200, and which is received by the communication I/F 60, and, by referring the information, determines whether the person to be measured is the registered user or not.

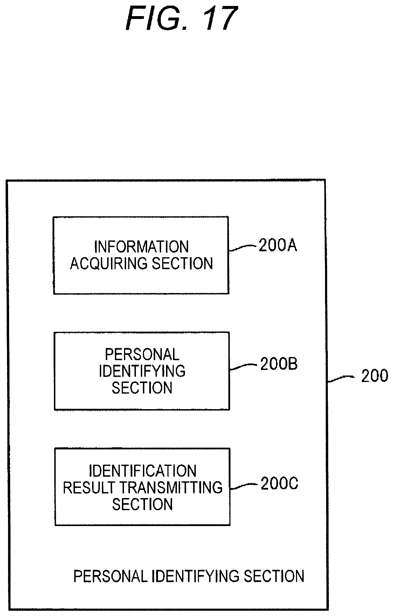

Fig. 17 is a functional block diagram of the personal identification device 200 shown in Fig. 14.

The personal identification device 200 includes an information acquiring section 200A, a personal identifying section 200B, and an identification result transmitting section 200C. The information acquiring section 200A, the personal identifying section 200B, and the identification result transmitting section 200C are formed by execution of a personal identification program stored in the ROM of the personal identification device 200, by a processor.

The information acquiring section 200A acquires the data of the tonogram which is transmitted from the biological information measurement device 100A, and causes the data to be stored in the RAM.

Based on the data of the tonogram which are acquired by the information acquiring section 200A, the personal identifying section 200B determines whether the person to be measured of a pressure signal contained in the data is the registered person or not, by using the method identical with the personal identification method which is performed by the personal identifying section 32 of the biological information measurement device 100.

In the personal authentication system 300, information of the registered user is previously stored in the ROM included in the personal identification device 200. Alternatively, a configuration where the biological information measurement device 100A transmits the information of the registered user together with the data of tonogram to the personal identification device 200 may be employed.

The identification result transmitting section 200C transmits information of a result (the person to be measured is the registered user, or the person to be measured is not the registered user) of the determination performed by the personal identifying section 200B, through the network 70 to the biological information measurement device 100A.

As described above, the configuration may be possible where an electronic apparatus other than the biological information measurement device 100A performs personal identification based on data which are produced from information of the pressure signals measured by the biological information measurement device 100A.

The above-described personal identification program is recorded on a non-transitory recording medium from which a computer can read the personal identification program.

Such "computer readable recording medium" includes an optical medium such as a CD-ROM (Compact Disc-ROM), a magnetic recording medium such as a memory card, and the like. Alternatively, such a program can be provided by downloading via a network.

The presently disclosed embodiments should be considered in all respects to be illustrative and not restrictive. The scope of the invention is indicated by the appended claims rather than the foregoing description, and all changes which come within the meaning and range of equivalents thereof are intended to be embraced therein.

As described above, the following matters are disclosed in the specification.

The disclosed biological information measurement device includes: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; a biological information calculating section which calculates biological information based on information of pressure pulse waves which are detected by the pressure detecting elements in a state where the pressure sensor is pressed against the wrist by the pressing mechanism; and a personal identifying section which determines whether the person to be measured is a registered user who is previously registered or not, based on data indicating relationships between information of pressure signals which are detected by the pressure detecting elements of the pressure sensor in a pressed state where the pressure sensor is pressed against the wrist by the pressing mechanism, and positions of the pressure detecting elements in the one direction.

In the disclosed biological information measurement device, the personal identifying section detects a first feature amount of a shape of a graph indicating the data while setting the information of the pressure signals which are detected by the pressure detecting elements in the pressed state where the pressure sensor is pressed against the wrist by the pressing mechanism, as a first axis, and the positions of the pressure detecting elements in the one direction, as a second axis, and determines whether the person to be measured is the registered user who is previously registered or not, based on a result of comparison between the first feature amount, and a second feature amount of the shape of the graph that is previously stored with respect to the registered user.

In the disclosed biological information measurement device, the first feature amount and the second feature amount are a number of a portion where the information of the pressure signals in the graph changes from increasing to decreasing, and a number of a portion where the information of the pressure signals in the graph changes from decreasing to increasing, respectively, and, in a case where the first feature amount and the second feature amount coincide with each other, the personal identifying section determines that the person to be measured is the registered user.

In the disclosed biological information measurement device, the first feature amount and the second feature amount are respectively one of: information of a distance between a first pressure detecting element and a second pressure detecting element, the first pressure detecting element corresponding to a point where the information of the pressure signals in the graph that is obtained in the pressed state where the pressure sensor is pressed against the wrist by the pressing mechanism changes from increasing to decreasing, the second pressure detecting element being located in an end portion on a side of a styloid process of a radius of the wrist in the pressed state; and information of a distance between a third pressure detecting element and the first pressure detecting element, the third pressure detecting element being one of pressure detecting elements corresponding to a point where the information of the pressure signals in the graph that is obtained in the pressed state changes from decreasing to increasing, and close to the side of the styloid process, and, in a case where a difference between the first feature amount and the second feature amount is smaller than a threshold, the personal identifying section determines that the person to be measured is the registered user.

In the disclosed biological information measurement device, the first feature amount contains a third feature amount and a fourth feature amount, the second feature amount contains a fifth feature amount and a sixth feature amount, the third feature amount and the fifth feature amount are a number of a portion where the information of the pressure signals in the graph changes from increasing to decreasing, and a number of a portion where the information of the pressure signals in the graph changes from decreasing to increasing, respectively, the fourth feature amount and the sixth feature amount are respectively one of: information of a distance between a first pressure detecting element and a second pressure detecting element, the first pressure detecting element corresponding to a point where the information of the pressure signals in the graph that is obtained in the pressed state where the pressure sensor is pressed against the wrist by the pressing mechanism changes from increasing to decreasing, the second pressure detecting element being located in an end portion on a side of a styloid process of a radius of the wrist in the pressed state; and information of a distance between a third pressure detecting element and the first pressure detecting element, the third pressure detecting element being one of pressure detecting elements corresponding to a point where the information of the pressure signals in the graph that is obtained in the pressed state changes from decreasing to increasing, and close to the side of the styloid process, and, in a case where the third feature amount and the fifth feature amount coincide with each other, and a difference between the fourth feature amount and the sixth feature amount is smaller than a threshold, the personal identifying section determines that the person to be measured is the registered user.

The disclosed biological information measurement device further includes a storage controller which controls storage of the biological information calculated by the biological information calculating section, in a storage medium, and, when the personal identifying section determines that the person to be measured is the registered user, the storage controller causes the biological information calculated by the biological information calculating section, to be stored in the storage medium, and, when the personal identifying section determines that the person to be measured is not the registered user, the storage controller causes the biological information calculated by the biological information calculating section, not to be stored in the storage medium.

The disclosed biological information measurement device further includes a storage controller which controls storage of the biological information calculated by the biological information calculating section, in a storage medium, and, when the personal identifying section determines that the person to be measured is the registered user, the storage controller causes the biological information calculated by the biological information calculating section, and information indicating the registered user, to be stored in the storage medium while associating with each other, and, when the personal identifying section determines that the person to be measured is not the registered user, the storage controller causes the biological information calculated by the biological information calculating section, and information indicating that the person to be measured is not the registered user, to be stored in the storage medium while associating with each other.

The disclosed personal identification device includes: an information acquiring section which acquires data from a biological information measurement device including: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; and a biological information calculating section which calculates biological information based on information of pressure pulse waves that are detected by the pressure detecting elements in a state where the pressure sensor is pressed against the wrist by the pressing mechanism, the data indicating relationships between information of pressure signals which are detected by the pressure detecting elements of the pressure sensor in a pressed state where the pressure sensor is pressed against the wrist by the pressing mechanism, and positions of the pressure detecting elements in the one direction; a personal identifying section which determines whether the person to be measured is a registered user who is previously registered or not, based on the data acquired by the information acquiring section; and a transmitting section which transmits a result of the determination performed by the personal identifying section, to the biological information measurement device.

The disclosed personal identification method is a method which is performed by a biological information measurement device including: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; and a biological information calculating section which calculates biological information based on information of pressure pulse waves that are detected by the pressure detecting elements in a state where the pressure sensor is pressed against the wrist by the pressing mechanism, the personal identification method including: a personal identifying step of determining whether the person to be measured is a registered user who is previously registered or not, based on data indicating relationships between information of pressure signals which are detected by the pressure detecting elements of the pressure sensor in a pressed state where the pressure sensor is pressed against the wrist by the pressing mechanism, and positions of the pressure detecting elements in the one direction.

The disclosed personal identification program is a program for causing a computer of a biological information measurement device including: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; and a biological information calculating section which calculates biological information based on information of pressure pulse waves that are detected by the pressure detecting elements in a state where the pressure sensor is pressed against the wrist by the pressing mechanism, to execute a personal identifying step of determining whether the person to be measured is a registered user who is previously registered or not, based on data indicating relationships between information of pressure signals which are detected by the pressure detecting elements of the pressure sensor in a pressed state where the pressure sensor is pressed against the wrist by the pressing mechanism, and positions of the pressure detecting elements in the one direction.

The disclosed personal identification method includes: an information acquiring step of acquiring data from a biological information measurement device including: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; and a biological information calculating section which calculates biological information based on information of pressure pulse waves that are detected by the pressure detecting elements in a state where the pressure sensor is pressed against the wrist by the pressing mechanism, the data indicating relationships between information of pressure signals which are detected by the pressure detecting elements of the pressure sensor in a pressed state where the pressure sensor is pressed against the wrist by the pressing mechanism, and positions of the pressure detecting elements in the one direction; a personal identifying step of determining whether the person to be measured is a registered user who is previously registered or not, based on the data acquired in the information acquiring step; and a transmitting step of transmitting a result of the determination performed in the personal identifying step, to the biological information measurement device.

The disclosed personal identification program is a program for causing a computer to execute: an information acquiring step of acquiring data from a biological information measurement device including: a pressure sensor which includes a plurality of pressure detecting elements which are arranged in one direction; a pressing mechanism which presses the pressure sensor against a wrist of a person to be measured, in a state where the one direction intersects with an elongation direction of a radial artery that is under a skin of the wrist; and a biological information calculating section which calculates biological information based on information of pressure pulse waves that are detected by the pressure detecting elements in a state where the pressure sensor is pressed against the wrist by the pressing mechanism, the data indicating relationships between information of pressure signals which are detected by the pressure detecting elements of the pressure sensor in a pressed state where the pressure sensor is pressed against the wrist by the pressing mechanism, and positions of the pressure detecting elements in the one direction; a personal identifying step of determining whether the person to be measured is a registered user who is previously registered or not, based on the data acquired in the information acquiring step; and a transmitting step of transmitting a result of the determination performed in the personal identifying step, to the biological information measurement device.

### Industrial Applicability

According to the invention, it is possible to provide a biological information measurement device, personal identification device, personal identification method, and personal identification program which can easily perform personal authentication at low cost regardless of the physical condition.

Although the invention has been described with reference to the specific embodiments, the invention is not limited to the embodiments, and various changes can be made without departing from the scope of the invention as solely defined by the appended claims.

- 100, 100A: biological information measurement device
- 1: body unit
- 2: belt
- 10: pressure sensor
- 20: pressing mechanism
- 11: substrate
- 12: pressure detecting element
- 120: element row
- 30, 30A: controller
- 31: pressing controller
- 32, 32A: personal identifying section
- 33: biological information calculating section
- 34: storage controller
- 40: storage medium
- 50: displaying section
- 60: communication interface
- 70: network
- 200: personal identification device
- 200A: information acquiring section
- 200B: personal identifying section
- 200C: identification result transmitting section
- 300: personal authentication system
- H: wrist
- K: tendon
- TB: radius
- TBa: styloid process
- TD: radial artery

## Claims

1. A biological information measurement device (100) comprising:
a pressure sensor (10) which includes a plurality of pressure detecting elements (12) which are arranged in one direction; a pressing mechanism (20) which is configured to press the pressure sensor against a wrist of a person to be measured, in a state where the one direction intersects with an elongation direction of a radial artery that is under a skin of the wrist;
a biological information calculating section (33) which is configured to calculate biological information based on information of pressure pulse waves which are detected by the pressure detecting elements (12) in a state where the pressure sensor (10) is pressed against the wrist by the pressing mechanism; and the device being **characterized by**:
a personal identifying section (32) which is configured to determine whether the person to be measured is a registered user who is previously registered or not, based on data indicating relationships between information of pressure signals which are detected by the pressure detecting elements (12) of the pressure sensor (10) in a pressed state where the pressure sensor (10) is pressed against the wrist by the pressing mechanism (20), and positions of the pressure detecting elements (12) in the one direction.

2. The biological information measurement device according to claim 1, wherein
the personal identifying section detects a first feature amount of a shape of a graph indicating the data while setting the information of the pressure signals which are detected by the pressure detecting elements in the pressed state where the pressure sensor is pressed against the wrist by the pressing mechanism, as a first axis, and the positions of the pressure detecting elements in the one direction, as a second axis, and determines whether the person to be measured is the registered user who is previously registered or not, based on a result of comparison between the first feature amount, and a second feature amount of the shape of the graph that is previously stored with respect to the registered user.

3. The biological information measurement device according to claim 2, wherein
the first feature amount and the second feature amount are a number of a portion where the information of the pressure signals in the graph changes from increasing to decreasing, and a number of a portion where the information of the pressure signals in the graph changes from decreasing to increasing, respectively, and,
in a case where the first feature amount and the second feature amount coincide with each other, the personal identifying section determines that the person to be measured is the registered user.

4. The biological information measurement device according to claim 2, wherein
the first feature amount and the second feature amount are respectively one of: information of a distance between a first pressure detecting element and a second pressure detecting element, the first pressure detecting element corresponding to a point where the information of the pressure signals in the graph that is obtained in the pressed state where the pressure sensor is pressed against the wrist by the pressing mechanism changes from increasing to decreasing, the second pressure detecting element being located in an end portion on a side of a styloid process of a radius of the wrist in the pressed state; and information of a distance between a third pressure detecting element and the first pressure detecting element, the third pressure detecting element being one of pressure detecting elements corresponding to a point where the information of the pressure signals in the graph that is obtained in the pressed state changes from decreasing to increasing, and close to the side of the styloid process, and,
in a case where a difference between the first feature amount and the second feature amount is smaller than a threshold, the personal identifying section determines that the person to be measured is the registered user.

5. The biological information measurement device according to claim 2, wherein
the first feature amount contains a third feature amount and a fourth feature amount,
the second feature amount contains a fifth feature amount and a sixth feature amount,
the third feature amount and the fifth feature amount are a number of a portion where the information of the pressure signals in the graph changes from increasing to decreasing, and a number of a portion where the information of the pressure signals in the graph changes from decreasing to increasing, respectively,
the fourth feature amount and the sixth feature amount are respectively one of: information of a distance between a first pressure detecting element and a second pressure detecting element, the first pressure detecting element corresponding to a point where the information of the pressure signals in the graph that is obtained in the pressed state where the pressure sensor is pressed against the wrist by the pressing mechanism changes from increasing to decreasing, the second pressure detecting element being located in an end portion on a side of a styloid process of a radius of the wrist in the pressed state; and information of a distance between a third pressure detecting element and the first pressure detecting element, the third pressure detecting element being one of pressure detecting elements corresponding to a point where the information of the pressure signals in the graph that is obtained in the pressed state changes from decreasing to increasing, and close to the side of the styloid process, and,
in a case where the third feature amount and the fifth feature amount coincide with each other, and a difference between the fourth feature amount and the sixth feature amount is smaller than a threshold, the personal identifying section determines that the person to be measured is the registered user.

6. The biological information measurement device according to any one of claims 1 to 5, further comprising: a storage controller which controls storage of the biological information calculated by the biological information calculating section, in a storage medium, wherein,
when the personal identifying section determines that the person to be measured is the registered user, the storage controller causes the biological information calculated by the biological information calculating section, to be stored in the storage medium, and, when the personal identifying section determines that the person to be measured is not the registered user, the storage controller causes the biological information calculated by the biological information calculating section, not to be stored in the storage medium.

7. The biological information measurement device according to any one of claims 1 to 5, further comprising: a storage controller which controls storage of the biological information calculated by the biological information calculating section, in a storage medium, wherein,
when the personal identifying section determines that the person to be measured is the registered user, the storage controller causes the biological information calculated by the biological information calculating section, and information indicating the registered user, to be stored in the storage medium while associating with each other, and, when the personal identifying section determines that the person to be measured is not the registered user, the storage controller causes the biological information calculated by the biological information calculating section, and information indicating that the person to be measured is not the registered user, to be stored in the storage medium while associating with each other.

8. A personal identification device (200) comprising: an information acquiring section (200A) which is configured to acquire data from a biological information measurement device (100A) including: a pressure sensor (10) which includes a plurality of pressure detecting elements (12) which are arranged in one direction; a pressing mechanism (20) which is configured to press the pressure sensor (10) against a wrist of a person to be measured, in a state where the one direction intersects with an elongation direction of a radial artery that is under a skin of the wrist; and a biological information calculating section (33) which is configured to calculate biological information based on information of pressure pulse waves that are detected by the pressure detecting elements (12) in a state where the pressure sensor (10) is pressed against the wrist by the pressing mechanism, the data indicating relationships between information of pressure signals which are detected by the pressure detecting elements (12) of the pressure sensor (10) in a pressed state where the pressure sensor (10) is pressed against the wrist by the pressing mechanism (20), and positions of the pressure detecting elements (12) in the one direction; and the device being **characterized by**:
a personal identifying section (200B) which is configured to determine whether the person to be measured is a registered user who is previously registered or not, based on the data acquired by the information acquiring section (200A); and
a transmitting section (200C) which is configured to transmit a result of the determination performed by the personal identifying section (200B), to the biological information measurement device (100A).

9. A personal identification method which is performed by a biological information measurement device (100) including: a pressure sensor (10) which includes a plurality of pressure detecting elements (12) which are arranged in one direction; a pressing mechanism (20) which presses the pressure sensor (10) against a wrist of a person to be measured, in a state where the one direction intersects with an elongation direction of a radial artery that is under a skin of the wrist; and a biological information calculating section (33) which calculates biological information based on information of pressure pulse waves that are detected by the pressure detecting elements (12) in a state where the pressure sensor (10) is pressed against the wrist by the pressing mechanism (20), the personal identification method being **characterized by**:
a personal identifying step of determining whether the person to be measured is a registered user who is previously registered or not, based on data indicating relationships between information of pressure signals which are detected by the pressure detecting elements (12) of the pressure sensor (10) in a pressed state where the pressure sensor (10) is pressed against the wrist by the pressing mechanism (20), and positions of the pressure detecting elements (12) in the one direction.

10. A personal identification program for causing a computer of a biological information measurement device including: a pressure sensor (10) which includes a plurality of pressure detecting elements (12) which are arranged in one direction; a pressing mechanism (20) which presses the pressure sensor (10) against a wrist of a person to be measured, in a state where the one direction intersects with an elongation direction of a radial artery that is under a skin of the wrist; and a biological information calculating section (33) which calculates biological information based on information of pressure pulse waves that are detected by the pressure detecting elements (12) in a state where the pressure sensor (10) is pressed against the wrist by the pressing mechanism (20), being characterized to
a personal identifying step of determining whether the person to be measured is a registered user who is previously registered or not, based on data indicating relationships between information of pressure signals which are detected by the pressure detecting elements (12) of the pressure sensor (10) in a pressed state where the pressure sensor (10) is pressed against the wrist by the pressing mechanism (20), and positions of the pressure detecting elements (12) in the one direction.

11. A personal identification method comprising:
an information acquiring step of acquiring data from a biological information measurement device (100A) including: a pressure sensor (10) which includes a plurality of pressure detecting (12) which are arranged in one direction; a pressing mechanism (20) which presses the pressure sensor (10) against a wrist of a person to be measured, in a state where the one direction intersects with an elongation direction of a radial artery that is under a skin of the wrist; and a biological information calculating section (33) which calculates biological information based on information of pressure pulse waves that are detected by the pressure detecting elements (12) in a state where the pressure sensor (10) is pressed against the wrist by the pressing mechanism (20), the data indicating relationships between information of pressure signals which are detected by the pressure detecting elements (12) of the pressure sensor (10) in a pressed state where the pressure sensor (10) is pressed against the wrist by the pressing mechanism (20), and positions of the pressure detecting elements (12) in the one direction; and the method being **characterized by**:
a personal identifying step of determining whether the person to be measured is a registered user who is previously registered or not, based on the data acquired in the information acquiring step; and
a transmitting step of transmitting a result of the determination performed in the personal identifying step, to the biological information measurement device (100A).

12. A personal identification program for causing a computer to execute:
an information acquiring step of acquiring data from a biological information measurement device (100A) including: a pressure sensor (10) which includes a plurality of pressure detecting elements (12) which are arranged in one direction; a pressing mechanism (20) which presses the pressure sensor (10) against a wrist of a person to be measured, in a state where the one direction intersects with an elongation direction of a radial artery that is under a skin of the wrist; and a biological information calculating section (33) which calculates biological information based on information of pressure pulse waves that are detected by the pressure detecting elements (12) in a state where the pressure sensor (10) is pressed against the wrist by the pressing mechanism (20), the data indicating relationships between information of pressure signals which are detected by the pressure detecting elements (12) of the pressure sensor (10) in a pressed state where the pressure sensor (10) is pressed against the wrist by the pressing mechanism (20), and positions of the pressure detecting elements (12) in the one direction; and the program being **characterized by**:
a personal identifying step of determining whether the person to be measured is a registered user who is previously registered or not, based on the data acquired in the information acquiring step; and
a transmitting step of transmitting a result of the determination performed in the personal identifying step, to the biological information measurement device (100A).

## Patentansprüche

1. Vorrichtung zum Messen biologischer Informationen (100), umfassend:
einen Drucksensor (10), der eine Vielzahl Druck erfassender Elemente (12) umfasst, die in einer Richtung angeordnet sind;
einen Druckmechanismus (20), der eingerichtet ist, um den Drucksensor gegen ein Handgelenk einer zu messenden Person zu drücken, in einem Zustand, in dem die eine Richtung eine Längsrichtung einer Radialarterie schneidet, die sich unter einer Haut des Handgelenks befindet;
einen Abschnitt zum Berechnen biologischer Informationen (33), der eingerichtet ist, um biologische Informationen basierend auf Informationen zu Druckpulswellen zu berechnen, die durch die Druck erfassenden Elemente (12) in einem Zustand erfasst werden, in dem der Drucksensor (10) durch den Druckmechanismus gegen das Handgelenk gedrückt wird; und
wobei die Vorrichtung **gekennzeichnet ist durch**:
einen Abschnitt zur Personenidentifikation (32), der eingerichtet ist, um zu bestimmen, ob die zu messende Person ein registrierter Nutzer ist, der zuvor registriert wurde, oder nicht, basierend auf Daten, die Beziehungen zwischen Informationen von Drucksignalen, die **durch** die Druck erfassenden Elemente (12) des Drucksensors (10) in einem gedrückten Zustand erfasst werden, in dem der Drucksensor (10) **durch** den Druckmechanismus (20) gegen das Handgelenk gedrückt wird, und Positionen der Druck erfassenden Elemente (12) in der einen Richtung anzeigen.

2. Vorrichtung zum Messen biologischer Informationen nach Anspruch 1, wobei
der Abschnitt zur Personenidentifikation eine erste Merkmalsmenge einer Form eines Graphen erfasst, der die Daten anzeigt, wobei die Informationen der Drucksignale, die durch die Druck erfassenden Elemente in dem gedrückten Zustand erfasst werden, in dem der Drucksensor durch den Druckmechanismus gegen das Handgelenk gedrückt wird, als eine erste Achse festgelegt werden, und die Positionen der Druck erfassenden Elemente in der einen Richtung als eine zweite Achse, und bestimmt, ob die zu messende Person der registrierte Nutzer ist, der zuvor registriert wurde, oder nicht, basierend auf einem Ergebnis eines Vergleichs zwischen der ersten Merkmalsmenge und einer zweiten Merkmalsmenge der Form des Graphen, die zuvor in Bezug auf den registrierten Nutzer gespeichert wurde.

3. Vorrichtung zum Messen biologischer Informationen nach Anspruch 2, wobei
die erste Merkmalsmenge und die zweite Merkmalsmenge jeweils eine Zahl eines Teils sind, in dem die Informationen der Drucksignale in dem Graphen sich von ansteigend zu absteigend verändern, und eine Zahl eines Teils, in dem die Informationen der Drucksignale in dem Graphen sich von absteigend zu ansteigend verändern, und
für den Fall, dass die erste Merkmalsmenge und die zweite Merkmalsmenge zusammentreffen, bestimmt der Abschnitt zur Personenidentifikation, dass die zu messende Person der registrierte Nutzer ist.

4. Vorrichtung zum Messen biologischer Informationen nach Anspruch 2, wobei
die erste Merkmalsmenge und die zweite Merkmalsmenge jeweils eines der Folgenden sind:
Informationen eines Abstands zwischen einem ersten Druck erfassenden Element und einem zweiten Druck erfassenden Element, wobei das erste Druck erfassende Element einem Punkt entspricht, an dem die Informationen der Drucksignale in dem Graphen, die in dem gedrückten Zustand erhalten werden, in dem der Drucksensor durch den Druckmechanismus gegen das Handgelenk gedrückt wird, sich von ansteigend zu absteigend ändern, wobei das zweite Druck erfassende Element sich in dem gedrückten Zustand in einem Endteil auf einer Seite eines Processus styloideus eines Radius des Handgelenks befindet; und
Informationen eines Abstands zwischen einem dritten Druck erfassenden Element und dem ersten Druck erfassenden Element, wobei das dritte Druck erfassende Element eines von Druck erfassenden Elementen ist, die einem Punkt entsprechen, an dem die Informationen der Drucksignale in dem Graphen, die in dem gedrückten Zustand erhalten werden, sich von absteigend zu ansteigend verändern, und das sich in der Nähe der Seite des Processus styloideus befindet, und für den Fall, dass ein Unterschied zwischen der ersten Merkmalsmenge und der zweiten Merkmalsmenge geringer als ein Grenzwert ist, der Abschnitt zur Personenidentifikation bestimmt, dass die zu messende Person der registrierte Nutzer ist.

5. Vorrichtung zum Messen biologischer Informationen nach Anspruch 2, wobei
die erste Merkmalsmenge eine dritte Merkmalsmenge und eine vierte Merkmalsmenge enthält,
die zweite Merkmalsmenge eine fünfte Merkmalsmenge und eine sechste Merkmalsmenge enthält,
die dritte Merkmalsmenge und die fünfte Merkmalsmenge jeweils eine Zahl eines Teils sind, an dem die Informationen der Drucksignale in dem Graphen sich von ansteigend zu absteigend verändern, und eine Zahl eines Teils, an dem die Informationen der Drucksignale in dem Graphen sich von absteigend zu ansteigend verändern,
die vierte Merkmalsmenge und die sechste Merkmalsmenge jeweils eines der Folgenden sind:
Informationen eines Abstands zwischen einem ersten Druck erfassenden Element und einem zweiten Druck erfassenden Element, wobei das erste Druck erfassende Element einem Punkt entspricht, an dem die Informationen der Drucksignale in dem Graphen, die in dem gedrückten Zustand erhalten werden, in dem der Drucksensor durch den Druckmechanismus gegen das Handgelenk gedrückt wird, sich von ansteigend zu absteigend verändern, wobei das zweite Druck erfassende Element sich in dem gedrückten Zustand in einem Endteil auf einer Seite eines Processus styloideus eines Radius des Handgelenks befindet; und
Informationen eines Abstands zwischen einem dritten Druck erfassenden Element und dem ersten Druck erfassenden Element, wobei das dritte Druck erfassende Element eines von Druck erfassenden Elementen ist, die einem Punkt entsprechen, an dem sich die Informationen der Drucksignale in dem Graph, die in dem gedrückten Zustand erhalten werden, sich von absteigend zu ansteigend verändern, und das sich in der Nähe des Processus styloideus befindet, und
für den Fall, dass die dritte Merkmalsmenge und die fünfte Merkmalsmenge zusammenfallen und ein Unterschied zwischen der vierte Merkmalsmenge und der sechsten Merkmalsmenge geringer als ein Grenzwert ist, der Abschnitt zur Personenidentifikation bestimmt, dass die zu messende Person der registrierte Nutzer ist.

6. Vorrichtung zum Messen biologischer Informationen nach einem der Ansprüche 1 bis 5, ferner umfassend: eine Speichersteuerung, welche die Speicherung der biologischen Informationen, die durch den Abschnitt zum Berechnen biologischer Informationen berechnet werden, in einem Speichermedium steuert, wobei,
wenn der Abschnitt zur Personenidentifikation bestimmt, dass die zu messende Person der registrierte Nutzer ist, die Speichersteuerung verursacht, dass die biologischen Informationen, die durch den Abschnitt zum Berechnen biologischer Informationen berechnet wurden, in dem Speichermedium gespeichert werden, und wenn der Abschnitt zur Personenidentifikation bestimmt, dass die zu messende Person nicht der registrierter Nutzer ist, die Speichersteuerung verursacht, dass die biologischen Informationen, die durch den Abschnitt zum Berechnen biologischer Informationen berechnet wurden, nicht auf dem Speichermedium gespeichert werden.

7. Vorrichtung zum Messen biologischer Informationen nach einem der Ansprüche 1 bis 5, ferner umfassend: eine Speichersteuerung, welche die Speicherung der biologischen Informationen, die durch den Abschnitt zum Berechnen biologischer Informationen berechnet wurden, auf einem Speichermedium steuert, wobei,
wenn der Abschnitt zur Personenidentifikation bestimmt, dass die zu messende Person der registrierte Nutzer ist, die Speichersteuerung verursacht, dass die biologischen Informationen, die durch den Abschnitt zum Berechnen biologischer Informationen berechnet wurden, und Informationen, die den registrierten Nutzer anzeigen, in dem Speichermedium gespeichert werden, während sie miteinander assoziiert werden, und wenn der Abschnitt zur Personenidentifikation bestimmt, dass die zu messende Person nicht der registrierte Nutzer ist, die Speichersteuerung verursacht, dass die biologischen Informationen, die durch den Abschnitt zum Berechnen biologischer Informationen berechnet wurden, und Informationen, die anzeigen, dass die zu messende Person nicht der registrierte Nutzer ist, auf dem Speichermedium gespeichert werden, während sie miteinander assoziiert werden.

8. Vorrichtung zur Personenidentifizierung (200), umfassend:
einen Informationen sammelnden Abschnitt (200A), der eingerichtet ist, um Daten von einer Vorrichtung zum Messen biologischer Informationen (100A) zu sammeln, die umfasst:
einen Drucksensor (10), der eine Vielzahl von Druck erfassenden Elementen (12) umfasst, die in einer Richtung angeordnet sind;
einen Druckmechanismus (20), der eingerichtet ist, um den Drucksensor (10) gegen ein Handgelenk einer zu messenden Person zu drücken, in einem Zustand, in dem die eine Richtung eine Längsrichtung einer Radialarterie schneidet, die sich unter einer Haut des Handgelenks befindet;
und einen Abschnitt zum Berechnen biologischer Informationen (33), der eingerichtet ist, um biologische Informationen basierend auf Informationen von Druckpulswellen zu berechnen, die durch die Druck erfassenden Elemente (12) in einem Zustand erfasst werden, in dem der Drucksensor (10) durch den Druckmechanismus gegen das Handgelenk gedrückt wird,
wobei die Daten Beziehungen zwischen Informationen von Drucksignalen, die durch die Druck erfassenden Elemente (12) des Drucksensors (10) in einem gedrückten Zustand erfasst werden, in dem der Drucksensor (10) durch den Druckmechanismus (20) gegen das Handgelenk gedrückt wird, und Positionen der Druck erfassenden Elemente (12) in der einen Richtung anzeigen; und wobei die Vorrichtung **gekennzeichnet ist durch**:
einen Abschnitt zur Personenidentifikation (200B), der eingerichtet ist, um zu bestimmen, ob die zu messende Person ein registrierter Nutzer ist, der zuvor registriert wurde, oder nicht, basierend auf den Daten, die **durch** den Informationen sammelnden Abschnitt (200A) gesammelt werden; und
einen übertragenden Abschnitt (200C), der eingerichtet ist, um ein Ergebnis der Bestimmung, die **durch** den Abschnitt zur Personenidentifikation (200B) durchgeführt wurde, an die Vorrichtung zum Messen biologischer Informationen (100A) zu übertragen.

9. Verfahren zur Personenidentifikation, das durch eine Vorrichtung zum Messen biologischer Informationen (100) durchgeführt wird, die umfasst:
einen Drucksensor (10), der eine Vielzahl Druck erfassender Elemente (12) umfasst, die in einer Richtung angeordnet sind;
einen Druckmechanismus (20), der den Drucksensor (10) gegen ein Handgelenk einer zu messenden Person drückt, in einem Zustand, in dem die eine Richtung eine Längsrichtung einer Radialarterie schneidet, die sich unter einer Haut des Handgelenks befindet;
und einen Abschnitt zum Berechnen biologischer Informationen (33), der biologische Informationen basierend auf Informationen von Druckpulswellen berechnet, die durch die Druck erfassenden Elemente (12) in einem Zustand erfasst werden, in dem der Drucksensor (10) durch den Druckmechanismus (20) gegen das Handgelenk gedrückt wird,
wobei das Verfahren zur Personenidentifikation **dadurch gekennzeichnet ist, dass**:
es einen Schritt der Personenidentifikation umfasst, bei dem bestimmt wird, ob die zu messende Person ein registrierter Nutzer ist, der zuvor registriert wurde, oder nicht,
basierend auf Daten, die Beziehungen zwischen Informationen von Drucksignalen, die durch die Druck erfassenden Elemente (12) des Drucksensors (10) in einem gedrückten Zustand erfasst werden, in dem der Drucksensor (10) durch den Druckmechanismus (20) gegen das Handgelenk gedrückt wird, und Positionen der Druck erfassenden Elemente (12) in der einen Richtung anzeigen.

10. Programm zur Personenidentifikation, um zu verursachen, dass ein Computer einer Vorrichtung zum Messen biologischer Informationen, die umfasst:
einen Drucksensor (10), der eine Vielzahl von Druck erfassenden Elementen (12) umfasst, die in einer Richtung angeordnet sind;
einen Druckmechanismus (20), der den Drucksensor (10) gegen ein Handgelenk einer zu messenden Person drückt, in einem Zustand, in dem die eine Richtung eine Längsrichtung einer Radialarterie schneidet, die sich unter einer Haut des Handgelenks befindet;
und einen Abschnitt zum Berechnen biologischer Informationen (33), der biologische Informationen basierend auf Informationen von Druckpulswellen berechnet, die durch die Druck erfassenden Elemente (12) in einem Zustand erfasst werden, in dem der Drucksensor (10) durch den Druckmechanismus (20) gegen das Handgelenk gedrückt wird,
**dadurch gekennzeichnet, dass** er Folgendes ausführt:
einen Schritt einer Personenidentifikation, wobei bestimmt wird, ob die zu messende Person ein registrierter Nutzer ist, der zuvor registriert wurde, oder nicht, basierend auf Daten, die Beziehungen zwischen Informationen von Drucksignalen, die durch die Druck erfassenden Elemente (12) des Drucksensor (10) in einem gedrückten Zustand erfasst werden, in dem der Drucksensor (10) durch den Druckmechanismus (20) gegen das Handgelenk gedrückt wird, und Positionen der Druck erfassenden Elemente (12) in der einen Richtung anzeigen.

11. Verfahren zur Personenidentifikation, umfassend:
einen Schritt des Sammelns von Informationen, bei dem Daten von einer Vorrichtung zum Messen biologischer Informationen (100A) gesammelt werden, die umfasst:
einen Drucksensor (10), der eine Vielzahl von Druck erfassenden Elementen (12) umfasst, die in einer Richtung angeordnet sind;
einen Druckmechanismus (20), der den Drucksensor (10) gegen das Handgelenk einer zu messenden Person drückt, in einem Zustand, in dem die eine Richtung eine Längsrichtung einer Radialarterie schneidet, die sich unter einer Haut des Handgelenks befindet;
und einen Abschnitt zum Berechnen biologischer Informationen (33), der biologische Informationen basierend auf Informationen von Druckpulswellen berechnet, die durch die Druck erfassenden Elemente (12) in einem Zustand erfasst werden, in dem der Drucksensor (10) durch den Druckmechanismus (20) gegen das Handgelenk gedrückt wird,
wobei die Daten Beziehungen von Drucksignalen, die durch die Druck erfassenden Elemente (12) des Drucksensors (10) in einem gedrückten Zustand erfasst werden, in dem der Drucksensor (10) durch den Druckmechanismus (20) gegen das Handgelenk gedrückt wird, und Positionen der Druck erfassenden Elemente (12) in der einen Richtung anzeigen und das Verfahren **gekennzeichnet ist durch**:
einen Schritt der Personenidentifikation, in dem bestimmt wird, ob die zu messende Person ein registrierter Nutzer ist, der zuvor registriert wurde, oder nicht, basierend auf Daten, die in dem Schritt des Sammelns von Informationen gesammelt wurden; und
einen Schritt des Übertragens eines Ergebnisses der Bestimmung, die in dem Schritt der Personenidentifikation durchgeführt wurde, an die Vorrichtung zum Messen biologischer Informationen (100A).

12. Programm zur Personenidentifikation, um zu bewirken, dass ein Computer Folgendes ausführt:
einen Schritt des Sammelns von Informationen, bei dem Daten von einer Vorrichtung zum Messen biologischer Informationen (100A) gesammelt werden, die umfasst:
einen Drucksensor (10), der eine Vielzahl von Druck erfassenden Elementen (12) umfasst, die in einer Richtung angeordnet sind;
einen Druckmechanismus (20), der den Drucksensor (10) gegen ein Handgelenk einer zu messenden Person drückt, in einem Zustand, in dem die eine Richtung eine Längsrichtung einer Radialarterie schneidet, die sich unter einer Haut des Handgelenks befindet;
und einen Abschnitt zum Berechnen biologischer Informationen (33), der biologische Informationen basierend auf Informationen von Pulsdruckwellen berechnet, die durch die Druck erfassenden Elemente (12) in einem Zustand erfasst werden, in dem der Drucksensor (10) durch den Druckmechanismus (20) gegen das Handgelenk gedrückt wird,
wobei die Daten Beziehungen zwischen Informationen von Drucksignalen, die durch die Druck erfassenden Elemente (12) des Drucksensors (10) in einem gedrückten Zustand erfasst werden, in dem der Drucksensor (10) durch den Druckmechanismus (20) gegen das Handgelenk gedrückt wird, und Positionen der Druck erfassenden Elemente (12) in der einen Richtung anzeigen; und das Programm ist **gekennzeichnet durch**:
einen Schritt der Personenidentifikation, in dem bestimmt wird, ob die zu messende Person ein registrierter Nutzer ist, der zuvor registriert wurde, oder nicht, basierend auf den Daten, die in dem Schritt des Sammelns von Informationen gesammelt wurden; und
einen Schritt des Übertragens, bei dem ein Ergebnis der Bestimmung, die in dem Schritt der Personenidentifikation durchgeführt wurde, an die Vorrichtung zum Messen biologischer Daten (100A) übertragen wird.

## Revendications

1. Dispositif de mesure d'informations biologiques (100) comprenant :
un capteur de pression (10) qui comprend une pluralité d'éléments de détection de pression (12) qui sont disposés dans une direction ;
un mécanisme de pression (20) qui est configuré pour presser le capteur de pression contre un poignet d'une personne à mesurer, dans un état où la une direction entre en intersection avec une direction d'élongation d'une artère radiale qui est sous une peau du poignet ;
une section de calcul d'informations biologiques (33) qui est configurée pour calculer les informations biologiques basées sur les informations d'ondes d'impulsion de pression qui sont détectées par les éléments de détection de pression (12) dans un état où le capteur de pression (10) est pressé contre le poignet par le mécanisme de pression ;
et le dispositif étant **caractérisé par** :
une section d'identification personnelle (32) qui est configurée pour déterminer si la personne à mesurer est un utilisateur enregistré qui a été préalablement enregistré ou pas, sur la base des données indiquant des relations entre les informations de signaux de pression qui sont détectés par les éléments de détection de pression (12) du capteur de pression (10) dans un état pressé où le capteur de pression (10) est pressé contre le poignet par le mécanisme de pression (20),
et les positions des éléments de détection de pression (12) dans une direction.

2. Dispositif de mesure d'informations biologiques selon la revendication 1, dans lequel
la section d'identification personnelle détecte une première quantité de caractéristique d'une forme d'un graphe indiquant les données tout en réglant les informations des signaux de pression qui sont détectés par les éléments de détection de pression dans l'état pressé où le capteur de pression est pressé contre le poignet par le mécanisme de pression, comme premier axe, et les positions des éléments de détection de pression dans une direction, comme second axe, et détermine si la personne à mesurer est l'utilisateur enregistré qui a été préalablement enregistré ou pas, sur la base d'un résultat de comparaison entre la première quantité de caractéristique et une seconde quantité de caractéristique de la forme du graphe qui a été préalablement stocké en rapport avec l'utilisateur enregistré.

3. Dispositif de mesure d'informations biologiques selon la revendication 2, dans lequel
la première quantité de caractéristique et la seconde quantité de caractéristique sont un nombre d'une partie où les informations des signaux de pression dans le graphe changent d'une augmentation à une diminution, et un nombre d'une partie où les informations des signaux de pression dans le graphe changent d'une diminution à une augmentation, respectivement, et,
dans un cas où la première quantité de caractéristique et la seconde quantité de caractéristique coïncident l'une avec l'autre, la section d'identification personnelle détermine que la personne à mesurer est l'utilisateur enregistré.

4. Dispositif de mesure d'informations biologiques selon la revendication 2, dans lequel
la première quantité de caractéristique et la seconde quantité de caractéristique sont respectivement une de :
les informations d'une distance entre un premier élément de détection de pression et un deuxième élément de détection de pression, le premier élément de détection de pression correspondant à un point où les informations des signaux de pression dans le graphe qui sont obtenues dans l'état pressé où le capteur de pression est pressé contre le poignet par le mécanisme de pression changent d'une augmentation à une diminution, le deuxième élément de détection de pression étant situé dans une partie d'extrémité sur un côté d'une apophyse styloïde d'un rayon du poignet dans l'état pressé ;
et les informations d'une distance entre un troisième élément de détection de pression et le premier élément de pression, le troisième élément de détection de pression étant un des éléments de détection de pression correspondant à un point où les informations des signaux de pression dans le graphe qui est obtenu dans l'état pressé changent d'une diminution à une augmentation, et proche du côté de l'apophyse styloïde, et,
dans un cas où une différence entre la première quantité de caractéristique et la seconde quantité de caractéristique est plus petite qu'un seuil, la section d'identification personnelle détermine que la personne à mesurer est l'utilisateur enregistré.

5. Dispositif de mesure d'informations biologiques selon la revendication 2, dans lequel
la première quantité de caractéristique contient une troisième quantité de caractéristique et une quatrième quantité de caractéristique,
la deuxième quantité de caractéristique contient une cinquième quantité de caractéristique et une sixième quantité de caractéristique,
la troisième quantité de caractéristique et la cinquième quantité de caractéristique sont un nombre d'une partie où les informations des signaux de pression dans le graphe changent d'une augmentation à une diminution, et un nombre d'une partie où les informations des signaux de pression dans le graphe changent d'une diminution à une augmentation, respectivement,
la quatrième quantité de caractéristique et la sixième quantité de caractéristique sont l'une de :
les informations d'une distance entre un premier élément de détection de pression et un deuxième élément de détection de pression, le premier élément de détection de pression correspondant à un point où les informations des signaux de pression dans le graphe qui est obtenu dans l'état pressé où le capteur de pression est pressé contre le poignet par le mécanisme de pression changent d'une augmentation à une diminution, le deuxième élément de détection de pression étant situé dans une partie d'extrémité sur un côté d'une apophyse styloïde d'un rayon du poignet dans l'état pressé ; et
les informations d'une distance entre un troisième élément de détection de pression et le premier élément de détection de pression, le troisième élément de détection de pression étant un des éléments de détection de pression à un point où les informations des signaux de pression dans le graphe qui est obtenu dans l'état pressé changent d'une diminution à une augmentation, et proche du côté de l'apophyse styloïde, et,
dans un cas où la troisième quantité de caractéristique et la cinquième quantité de caractéristique coïncident l'une avec l'autre, et une différence entre la quatrième quantité de caractéristique et la sixième quantité de caractéristique est plus petite qu'un seuil, la section d'identification personnelle détermine que la personne à mesurer est l'utilisateur enregistré.

6. Dispositif de mesure d'informations biologiques selon l'une quelconque des revendications 1 à 5, comprenant en outre :
un contrôleur de stockage qui contrôle le stockage des informations biologiques calculées par la section de calcul d'informations biologiques, dans un support de stockage, lorsque la section d'identification personnelle détermine que la personne à mesurer est l'utilisateur enregistré, le contrôleur de stockage entraînant les informations biologiques calculées par la section de calcul d'informations biologiques, à être stockées dans le support de stockage, et, lorsque la section d'identification personnelle détermine que la personne à mesurer n'est pas l'utilisateur enregistré, le contrôleur de stockage entraînant les informations biologiques calculées par la section de calcul d'informations biologiques, à ne pas être stockées dans le support de stockage.

7. Dispositif de mesure d'informations biologiques selon l'une quelconque des revendications 1 à 5, comprenant en outre :
un contrôleur de stockage qui contrôle le stockage des informations biologiques calculées par la section de calcul d'informations biologiques, dans un support de stockage, lorsque la section d'identification personnelle détermine que la personne à mesurer est l'utilisateur enregistré, le contrôleur de stockage entraînant les informations biologiques calculées par la section de calcul d'informations biologiques, et les informations indiquant l'utilisateur enregistré, à être stockées dans le support de stockage tout en s'associant les unes avec les autres, et, lorsque la section d'identification personnelle détermine que la personne à mesurer n'est pas l'utilisateur enregistré, le contrôleur de stockage entraînant les informations biologiques calculées par la section de calcul des informations biologiques, et les informations indiquant que la personne à mesurer n'est pas l'utilisateur enregistré, à être stockées dans le support de stockage tout en s'associant les unes avec les autres.

8. Dispositif d'identification personnelle (200) comprenant :
une section d'acquisition d'informations (200A) qui est configurée pour acquérir les données d'un dispositif de mesure d'informations biologiques (100A) comprenant : un capteur de pression (10) qui comprend une pluralité d'éléments de détection de pression (12) qui sont disposés dans une direction ; un mécanisme de pression (20) qui est configuré pour presser le capteur de pression (10) contre un poignet d'une personne à mesurer, dans un état où la une direction entre en intersection avec une direction d'élongation d'une artère radiale qui est sous une peau du poignet ; et une section de calcul d'informations biologiques (33) qui est configurée pour calculer les informations biologiques basées sur les informations des ondes d'impulsion de pression qui sont détectées par les éléments de détection de pression (12) dans un état où le capteur de pression (10) est pressé contre le poignet par le mécanisme de pression, les données indiquant des relations entre les informations de signaux de pression qui sont détectés par les éléments de détection de pression (12) du capteur de pression (10) dans un état pressé où le capteur de pression (10) est pressé contre le poignet par le mécanisme de pression (20), et les positions des éléments de détection de pression (12) dans la une direction ; et le dispositif étant **caractérisé par** :
une section d'identification personnelle (200B) qui est configurée pour déterminer si la personne à mesurer est un utilisateur enregistré qui a été préalablement enregistré ou pas, sur la base des données acquises par la section d'acquisition d'informations (200A) ; et
une section de transmission (200C) qui est configurée pour transmettre un résultat de la détermination effectuée par la section d'identification personnelle (200B),
au dispositif de mesure des informations biologiques (100A).

9. Procédé d'identification personnelle qui est effectuée par un dispositif de mesure d'informations biologiques (100) comprenant : un capteur de pression (10) qui comprend une pluralité d'éléments de détection de pression (12) qui sont disposés dans une direction ; un mécanisme de pression (20) qui presse le capteur de pression (10) contre un poignet d'une personne à mesurer, dans un état où la une direction entre en intersection avec une direction d'élongation d'une artère radiale qui est sous une peau du poignet ; et une section de calcul d'informations biologiques (33) qui calcule les informations biologiques sur la base des informations d'ondes d'impulsion de pression qui sont détectées par les éléments de détection de pression (12) dans un état où le capteur de pression (10) est pressé contre le poignet par le mécanisme de pression (20), le procédé d'identification personnelle étant **caractérisé par** :
une étape d'identification personnelle de détermination si la personne à mesurer est un utilisateur enregistré qui a été préalablement enregistré ou pas, sur la base des données indiquant des relations entre les informations de signaux de pression qui sont détectés par les éléments de détection de pression (12) du capteur de pression (10) dans un état pressé où le capteur de pression (10) est pressé contre le poignet par le mécanisme de pression (20), et les positions des éléments de détection de pression (12) dans une direction.

10. Programme d'identification personnelle pour entraîner un ordinateur d'un dispositif de mesure d'informations biologiques comprenant : un capteur de pression (10) qui comprend une pluralité d'éléments de capture de pression (12) qui sont disposés dans une direction ; un mécanisme de pression (20) qui presse le capteur de pression (10) contre un poignet d'une personne à mesurer, dans un état où la une direction entre en intersection avec une direction d'élongation d'une artère radiale qui est sous une peau du poignet ; et une section de calcul d'informations biologiques (33) qui calcule les informations biologiques basées sur les informations d'ondes d'impulsion de pression qui sont détectées par les éléments de détection de pression (12) dans un état où le capteur de pression (10) est pressé contre le poignet par le mécanisme de pression (20), étant caractérisé pour
une étape d'identification personnelle de détermination si la personne à mesurer est un utilisateur enregistré qui a été préalablement enregistré ou pas, sur la base des données indiquant des relations entre les informations de signaux de pression qui sont détectés par les éléments de détection de pression (12) du capteur de pression (10) dans un état pressé où le capteur de pression (10) est pressé contre le poignet par le mécanisme de pression (20), et les positions des éléments de détection de pression (12) dans la une direction.

11. Procédé d'identification personnelle comprenant :
une étape d'acquisition d'informations à partir d'un dispositif de mesure d'informations biologiques (100A) comprenant : un capteur de pression (10) qui comprend une pluralité d'éléments de détection de pression qui sont disposés dans une direction ; un mécanisme de pression (20) qui presse le capteur de pression (10) contre un poignet d'une personne à mesurer, dans un état où la une direction entre en intersection avec une direction d'élongation d'une artère radiale qui est sous une peau du poignet ; et une section de calcul d'informations biologiques (33) qui calcule les informations biologiques sur la base des informations d'ondes d'impulsion de pression qui sont détectées par les éléments de détection de pression (12) dans un état où le capteur de pression (10) est pressé contre le poignet par le mécanisme de pression (20), les données indiquant des relations entre les informations de signaux de pression qui sont détectés par les éléments de détection de pression (12) du capteur de pression (10) dans un état pressé où le capteur de pression (10) est pressé contre le poignet par le mécanisme de pression (20), et les positions des éléments de détection de pression (12) dans la une direction ; et le procédé étant **caractérisé par** :
une étape d'identification personnelle de détermination si la personne à mesurer est un utilisateur enregistré qui a été préalablement enregistré ou pas, sur la base des données acquises dans l'étape d'acquisition d'informations ; et
une étape de transmission de transmission d'un résultat de la détermination effectuée dans l'étape d'identification personnelle, au dispositif de mesure d'informations biologiques (100A).

12. Programme d'identification personnelle pour entraîner un ordinateur à exécuter :
une étape d'acquisition d'informations d'acquisition de données d'un dispositif de mesure d'informations biologiques (100A) comprenant :
un capteur de pression (10) qui comprend une pluralité d'éléments de détection de pression (12) qui sont disposés dans une direction ; un mécanisme de pression (20) qui presse le capteur de pression (10) contre un poignet d'une personne à mesurer, dans un état où la une direction entre en intersection avec une direction d'élongation d'une artère radiale qui est sous une peau du poignet ; et une section de calcul d'informations biologiques (33) qui calcule les informations biologiques sur la base des informations d'ondes d'impulsion de pression qui sont détectées par les éléments de détection de pression (12) dans un état où le capteur de pression (10) est pressé contre le poignet par le mécanisme de pression (20), les données indiquant des relations entre des informations de signaux de pression qui sont détectés par les éléments de détection de pression (12) du capteur de pression (10) dans un état pressé où le capteur de pression (10) est pressé contre le poignet par le mécanisme de pression (20), et les positions des éléments de détection de pression (12) dans la une direction ; et le programme étant **caractérisé par** :
une étape d'identification personnelle de détermination si la personne à mesurer est un utilisateur enregistré qui a été préalablement enregistré ou pas, sur la base des données acquises dans l'étape d'acquisition d'informations ; et
une étape de transmission de transmission d'un résultat de la détermination effectuée dans l'étape d'identification personnelle, au dispositif de mesure d'informations biologiques (100A).
